# FASCICULE DE BREVET EUROPEEN

(11) **EP 3 899 000 B1**
(45) Date de publication et mention de la délivrance du brevet: **13.08.2025**
(21) Numéro de dépôt: 19829471.2
(22) Date de dépôt: 13.12.2019
(51) Int. Cl.: C12P 7/10, C08H 8/00

(54) **PROCÉDÉ DE TRAITEMENT DE BIOMASSE LIGNOCELLULOSIQUE**
VERFAHREN ZUR BEHANDLUNG VON LIGNOCELLULOSEHALTIGER BIOMASSE
PROCESS FOR TREATING LIGNOCELLULOSIC BIOMASS

(30) Priorité: 21.12.2018 FR 1873761
(43) Date de publication de la demande: 27.10.2021
(73) Titulaire: IFP Energies nouvelles, 92852 Rueil-Malmaison (FR); Institut national de recherche pour l'agriculture, l'alimentation et l'environnement, 75338 Paris Cedex 07 (FR); Agro Industries Recherche Et Developpement, 51110 Pomacle (FR)
(72) Inventeur: BOURAS, Meriem, 92852 RUEIL-MALMAISON (FR); AYMARD, Caroline, 92852 RUEIL-MALMAISON (FR); ARAGONES, Matthieu, 92852 RUEIL-MALMAISON (FR)
(74) Mandataire: IFP Energies nouvelles
(86) Numéro de dépôt international: PCT/EP2019/085108
(87) Numéro de publication internationale: WO 2020/126917

(56) Documents cités:
- WO-A1-2016/066752
- FR-A1- 3 043 408

## Description

### Domaine technique

L'invention concerne un procédé de traitement de biomasse lignocellulosique pour produire des jus sucrés dits de seconde génération (« 2G »). Ces jus sucrés peuvent être utilisés pour produire d'autres produits par voie biochimique (par exemple des alcools comme l'éthanol, le butanol ou d'autres molécules, ou des solvants tels que l'acétone etc...).

Ce procédé comporte différentes étapes, dont généralement trois étapes qui sont la préparation de liqueur, l'imprégnation de la biomasse par cette liqueur, et le prétraitement de la biomasse imprégnée par cuisson, notamment avec explosion à la vapeur.

### Technique antérieure

La biomasse lignocellulosique représente une des ressources renouvelables les plus abondantes sur terre. Les substrats considérés sont très variés, ils concernent à la fois des substrats ligneux comme différents bois (feuillus et résineux), des coproduits issus de l'agriculture (pailles de blé, rafles de maïs, etc...) ou d'autres industries agroalimentaires, papetières, etc...

Le procédé de transformation biochimique de la lignocellulosique en jus sucrés 2G comprend notamment une étape de prétraitement et une étape d'hydrolyse enzymatique par un cocktail enzymatique. Ces procédés comportent aussi le plus souvent une étape d'imprégnation avant le prétraitement. Les jus sucrés issus de l'hydrolyse sont ensuite traités, par exemple par fermentation, et le procédé peut comprendre également des étapes de séparation et/ou une étape de purification du produit final.

Ces procédés comportent aussi le plus souvent une étape d'imprégnation avant le prétraitement. Les jus sucrés issus de l'hydrolyse sont ensuite traités, par exemple par fermentation, et le procédé peut comprendre également des étapes de séparation et/ou une étape de purification du produit final.

Un exemple de cette succession d'étapes permettant de convertir de la biomasse en éthanol est décrit dans le brevet WO 2014/135755.

La biomasse lignocellulosique est composée de trois principaux polymères : la cellulose (35 à 50%), qui est un polysaccharide essentiellement constitué d'hexoses ; l'hémicellulose (20 à 30%), qui est un polysaccharide essentiellement constitué de pentoses ; et la lignine (15 à 25%), qui est un polymère de structure complexe et de haut poids moléculaire, composé d'alcools aromatiques reliés par des liaisons éther. Ces différentes molécules sont responsables des propriétés intrinsèques de la paroi végétale et s'organisent en un enchevêtrement complexe.

Parmi les trois polymères de base qui intègrent la biomasse lignocellulosique, la cellulose et l'hémicellulose sont ceux qui permettent la production de jus sucrés 2G.

Le plus souvent, l'hémicellulose est majoritairement décomposée en sucres monomères et oligomères durant le prétraitement, et la cellulose est convertie en glucose par l'hydrolyse enzymatique. Toutefois, l'accès à la cellulose brute dans le substrat natif reste difficilement accessibles aux enzymes, d'où la nécessité d'un prétraitement. Ce prétraitement permet de modifier les propriétés physico-chimiques de la lignocellulosique afin d'améliorer l'accessibilité de la cellulose aux enzymes et sa réactivité à l'hydrolyse enzymatique.

De nombreuses technologies intéressant l'invention pour réaliser ce prétraitement existent, qui seront ci-après regroupées sous le terme générique de « cuisson », consistent à chauffer la biomasse à haute température pendant une durée définie. On connaît notamment les cuissons acides, où la biomasse est mise en contact avec une solution acide avant/pendant la cuisson, les cuissons alcalines, où la biomasse est mise en contact avec une solution basique avant/pendant la cuisson. On connait aussi la cuisson (acide, alcaline ou sans imprégnation) dite par explosion à la vapeur, où la biomasse est soumise à de la vapeur d'eau sous pression.

Il existe aussi des procédés de prétraitement dits « organosolv pulping » selon le terme anglais connu (ou traitement avec organo-solvent en français). Ce dernier procédé concerne un prétraitement en présence d'un ou plusieurs solvants organiques et généralement d'eau. Le solvant peut être un alcool (éthanol), un acide type acide acétique, acide formique, ou encore l'acétone, ou encore un mélange de ces composés. Les procédés « organosolv pulping » conduisent à une solubilisation au moins partielle de la lignine, une solubilisation partielle des hémicelluloses. On a alors deux flux en sortie : le substrat prétraité avec cellulose, hémicellulose et lignine résiduels et la phase solvant qui contient la ligne solubilisée et une partie des hémicellulose. Il y a généralement une étape de régénération du solvant qui permet d'extraire un flux de lignine. Certains traitements « organosolv pulping » (notamment avec de l'éthanol) peuvent être couplés avec l'ajout d'un acide fort (du type H₂SO₄). On peut envisager également d'avoir une mise en contact de la biomasse avec le solvant via un réacteur d'imprégnation avant la phase de cuisson ou d'avoir une mise en contact de la biomasse avec le catalyseur acide avant d'effectuer une cuisson « organosolv pulping ».

Différentes configurations sont reportées par exemple dans le document «Production of bioethanol from lignocellulosic materials via the biochemical pathway: A review », M. Balat, Energy Conversion and Management 52 (2011) 858-875, ou encore dans le document « Bioethanol production from agricultural wastes : an overview », N. Sarkar, S. Kumar Ghosh, S. Bannerjee, K. Aikat, Renwable Energy 37 (2012) 19-27.

WO 2016/066752 A1 divulgue un procédé de traitement ignocellulosique en vue de produire des sucres et leurs produits de fermentation. Il aborde le problème d'encrassement et de blocage des éléments de l'installation utilisée pour le procédé de traitement lignocellulosique. Le procédé comprend les étapes suivantes: une étape de prétraitement de la biomasse comprenant deux étapes correspondant aux étapes de prétraitement selon la présente demande ; une étape de fermentation du produit prétraité, pouvant inclure une étape d'hydrolyse enzymatique. Le procédé est caractérise en ce que le produit issu de l'étape de prétraitement est mélange avec une partie du produit obtenu a l'étape de fermentation avant d'être introduit dans la cuve de fermentation.

Un des prétraitements les plus efficaces est la cuisson avec explosion à la vapeur mentionnée plus haut, car elle permet une hydrolyse presque totale de l'hémicellulose et une amélioration importante de l'accessibilité et la réactivité de la cellulose aux enzymes. Ce prétraitement peut être précédé/suivi d'autre(s) traitement(s), et c'est celui qui intéressera plus particulièrement l'invention, sans y être limité dans son application cependant.

Il a été mis en évidence que les réacteurs de traitement de la biomasse lignocellulosique, notamment ceux concernant le prétraitement de la biomasse de type par cuisson, étaient enclins à l'encrassement : chauffer le milieu réactionnel dans le réacteur à haute température conduit à la production de différents résidus solides qui viennent adhérer aux parois internes du réacteur. Ces résidus s'accumulent au fur et à mesure, sur un temps plus long que le temps de séjour moyen de la biomasse dans le réacteur. Ils peuvent progressivement engendrer des problèmes opératoires, comme par exemple un risque de bouchage de la sortie du réacteur, une plus grande difficulté à transporter la biomasse au sein du réacteur, et, de manière générale, leur présence impacte négativement les performances du réacteur. Une première solution apportée pour supprimer ces résidus a consisté à vider le réacteur, à l'arrêter donc, et à l'ouvrir, pour opérer un nettoyage des parois internes du réacteur par action mécanique et/ou hydraulique, c'est-à-dire par grattage des parois et/ou par envoi d'eau sous pression à plusieurs centaines de bars. Cette procédure est certes efficace, mais elle est consommatrice de temps et d'énergie : elle contraint non seulement à arrêter le réacteur, mais aussi à attendre son refroidissement (la cuisson se fait à une température supérieure à 100°C et sous pression), puis à l'ouvrir, à opérer le nettoyage, à refermer le réacteur et à le remettre en température avant de relancer la production.

L'invention a alors pour but de remédier à ces inconvénients. Plus précisément, l'invention a pour but de mettre au point un procédé de traitement amélioré de la biomasse qui élimine, en tout ou partie, ces résidus, ou qui en prévient, en tout ou partie, la formation, tout en limitant toute perte de rendement / toute immobilisation des équipements mettant en œuvre le procédé en question dans son ensemble.

### Résumé de l'invention

L'invention a tout d'abord pour objet un procédé de traitement de biomasse lignocellulosique comprenant, successivement,
b) une étape de prétraitement par cuisson, avec ou sans explosion à la vapeur de la biomasse préalablement mise dans des conditions acides ou à pH neutre dans un réacteur de prétraitement (3), pour produire un marc prétraité acide (MA) ou neutre,
en alternance avec
b') une étape de prétraitement par cuisson, avec ou sans explosion à la vapeur de la biomasse préalablement mise dans des conditions acide, neutre ou basique, avec introduction suffisante dans le réacteur de prétraitement (3) d'une solution aqueuse basique (EB) au moins pour la biomasse préalablement mise en condition acide ou neutre, pour produire un marc prétraité basique (MB),
   puis
c) une étape d'hydrolyse enzymatique dans un réacteur d'hydrolyse (16) d'un mélange du marc prétraité acide (MA) ou neutre issu de l'étape b) avec le marc prétraité basique (MB) issu de l'étape b').

Avantageusement, pendant l'étape c) d'hydrolyse enzymatique du mélange de marc prétraité acide (MA) ou neutre avec le marc prétraité basique, on peut remplacer ledit mélange par seulement du marc prétraité acide ou neutre pendant une partie seulement de la durée de ladite l'étape c).

On comprend par « successivement », le fait que les étapes sont énoncées selon l'ordre avec lequel la biomasse est traitée et chemine dans l'installation, sans préjuger du fait qu'il puisse y avoir dans le procédé des étapes intermédiaires, préliminaires ou postérieures à celles énoncées.

L'invention a ainsi découvert qu'on parvenait à nettoyer et/ou à prévenir l'encrassement du réacteur de prétraitement selon l'invention, en mettant en contact la biomasse avec une solution aqueuse basique (qui pourra aussi être désignée par la suite par le terme « liqueur » basique), par exemple une solution contenant du KOH, pendant le prétraitement même de la biomasse : le nettoyage se fait ainsi en maintenant l'alimentation en biomasse du réacteur.

Le nettoyage du réacteur est donc effectué avec un réacteur au moins partiellement rempli de biomasse, qui a pu, préalablement à son prétraitement dans le réacteur en question, être pré imprégnée d'une solution aqueuse acide, neutre ou oxydante.

La solution basique peut déjà être présente dans la biomasse quand elle a été préalablement imprégnée par une solution basique avant son introduction dans le réacteur de prétraitement, on peut ensuite seulement, au niveau de ce réacteur, faire l'appoint nécessaire en base, le cas échéant. Si la biomasse qui alimente le réacteur de prétraitement n'est pas basique, alors l'ajout de solution basique dans le réacteur de prétraitement sera indispensable.

Le nettoyage selon l'invention se fait par deux actions conjuguées : il est d'abord chimique, avec l'action de la solution basique qui s'est révélée apte à décoller et/ou dissoudre les résidus solides collant aux parois internes du réacteur, mais il est également mécanique, les particules de biomasse exerçant un effet abrasif, en plus, sur lesdits résidus.

C'est un procédé nettement plus simple à mettre en œuvre qu'un nettoyage mécanique ou hydraulique : en effet, il peut s'effectuer sans avoir à ouvrir le réacteur, puisqu'il suffit de prévoir les arrivées/sorties de solution aqueuse basique (ou de réutiliser des arrivées/sorties de fluide déjà présentes).

Il s'effectue sans même avoir à arrêter la production en biomasse prétraitée : de façon surprenante, il s'est en effet avéré que la présence de biomasse avait un effet abrasif, donc positif vis-à-vis du nettoyage du réacteur comme mentionné plus haut, mais surtout il s'est également avéré que la biomasse qui se trouve dans le réacteur avec la solution basique était exploitable/valorisable : le prétraitement s'effectue également correctement pendant la phase de nettoyage, la biomasse est utilisable pour la suite du procédé, et ceci même si elle a subi avant son prétraitement une imprégnation en solution acide ou neutre ou oxydante.

En outre, contrairement au nettoyage mécanique/hydraulique avec ouverture du réacteur, il n'est pas nécessaire d'attendre le refroidissement du réacteur pour opérer le nettoyage. Au contraire, il s'est même avéré qu'effectuer le nettoyage pendant le prétraitement à chaud, du type cuisson (avec ou sans explosion à la vapeur) était très favorable au décollement des résidus. Le réacteur pouvant rester chaud pendant le nettoyage, il n'y a pas besoin de remise en température de celui-ci, puisqu'il n'y a pas d'arrêt de production, donc pas de redémarrage non plus à prévoir sur le plan thermique.

Par contre, il s'est avéré que le marc prétraité basique, celui obtenu à l'étape b'), réagissait nettement moins bien à l'hydrolyse enzymatique suivante que le marc prétraité acide ou neutre conventionnel obtenu à l'étape b) lorsqu'il est utilisé seul. Le rendement de l'hydrolyse, ou le taux de conversion en alcool lorsque l'hydrolyse est concomitante à une fermentation, sont moins bons. Considérer, par ailleurs, que ce marc prétraité « basique » devait être éliminé et ne pouvait pas être valorisé aurait conduit aussi à une perte globale de rendement du procédé de traitement de la biomasse.

L'invention a alors surmonté cet obstacle, en prévoyant d'alimenter le réacteur d'hydrolyse non pas alternativement avec du marc basique et du marc acide/neutre, selon que le prétraitement se fait selon l'étape b) ou b'), (ni en « supprimant » le marc basique), mais en l'alimentant avec un mélange de marc acide ou neutre « conventionnel » avec ce marc basique.

On peut réaliser ce mélange de plusieurs manières, soit en prévoyant une unique zone tampon de marc basique, qu'on vient ensuite prélever pour l'additionner au marc acide/neutre conventionnel, soit en prévoyant deux zones tampons, l'une pour le marc basique, l'autre pour le marc acide/neutre, zones dont on vient prélever des quantités données pour avoir le mélange dans les proportions voulues dans le réacteur d'hydrolyse enzymatique.

Il est à noter que l'hydrolyse enzymatique peut être réalisée soit, en continu, par alimentation du réacteur d'hydrolyse depuis le réacteur de prétraitement, soit en « batch », pas en continu donc, et le choix de la façon dont on opère le mélange et le nombre de zones tampon va dépendre de ce mode de fonctionnement également.

Dans le cas de figure avec deux zones tampons, une pour le marc acide/neutre conventionnel et une pour le marc basique, on choisit de préférence des temps de rétention différents pour le marc acide/neutre et pour le marc basique. Ces zones tampons permettent ainsi de découpler la production de marc prétraité lors de l'étape de prétraitement de l'alimentation en marcs de l'étape avale d'hydrolyse enzymatique. Il devient ainsi possible d'alimenter l'hydrolyse enzymatique uniquement avec un mélange de marc acide/neutre avec du marc basique, éventuellement interrompue par une alimentation qu'avec du marc acide/neutre conventionnel quand le marc basique a été entièrement consommé. De façon surprenante, ce mode de fonctionnement a permis de préserver le rendement de l'hydrolyse enzymatique par rapport à une hydrolyse faite 100% à partir de marc acide/neutre, en ajustant les ratios des deux types de biomasse dans le mélange. On ajuste ainsi le pH du mélange, notamment à un niveau plus proche du niveau le plus adapté au cocktail enzymatique utilisé, généralement à un pH un peu supérieur au pH des marcs prétraités acides conventionnels (bien que restant acide).

L'invention permet donc de garantir une durée de fonctionnement plus longue du réacteur de prétraitement en ralentissant son encrassement, sans pour autant impacter négativement le rendement des étapes suivant le prétraitement, notamment l'hydrolyse enzymatique, alors qu'on aurait pu croire que le marc prétraité « basique » ne serait pas valorisable ou viendrait perturber ces étapes suivantes, et ceci en combinant deux caractéristiques : un prétraitement qui bascule périodiquement à pH basique plutôt qu'à pH acide ou neutre, associé à une conduite spécifique de l'alimentation en marc prétraité (acide/neutre ou basique) de l'hydrolyse enzymatique s'adaptant à ce marc basique.

Il est à noter, naturellement, que, en sortie du réacteur de prétraitement, quand on bascule de l'étape b) en conditions acide/neutre à l'étape b') en conditions basiques dans le réacteur de prétraitement, le marc prétraité en sortie du réacteur ne bascule pas brusquement d'un marc acide/neutre à un marc basique ou moins acide : pendant une certaine durée, on soutire du réacteur de prétraitement un marc dit « de transition » dont le pH augmente progressivement. De même, quand on rebascule d'un prétraitement en conditions basiques de type b') vers un prétraitement en conditions acide/neutre de type b), on a à nouveau pendant une certaine durée en sortie du réacteur un autre marc dit « de transition », dont le pH, cette fois, descend progressivement. On peut donc choisir arbitrairement, dans chacun des cas, une valeur seuil, notamment de pH ou représentative du pH, de la biomasse prétraitée en sortie du réacteur de prétraitement pour considérer si on a affaire à un marc de type acide/neutre ou un marc de type basique.

Avantageusement, le mélange du marc prétraité acide ou neutre issu de l'étape b) avec le marc prétraité basique issu de l'étape b') dans l'étape c) est réalisé en amont du réacteur d'hydrolyse, ou directement dans le réacteur d'hydrolyse.

Dans le premier cas de figure, on peut ainsi réaliser le mélange, dans l'étape c), du marc prétraité acide ou neutre issu de l'étape b) avec le marc prétraité basique issu de l'étape b') par prélèvements à partir d'une ou de plusieurs zones tampon, comme évoqué plus haut. Ces zones tampon sont des zones de stockage temporaire, qui peuvent prendre la forme de cuves de stockage ou d'autres moyens de stockage, comme une trémie.

Comme expliqué plus haut, l'étape b') de prétraitement de la biomasse avec introduction suffisante, le cas échéant, dans le réacteur de prétraitement d'une solution aqueuse basique (EB), pour produire un marc prétraité basique est une étape de nettoyage du réacteur de prétraitement, tout en continuant à produire du marc, aux caractéristiques, notamment de pH, différentes cependant.

De préférence, on prévoit une étape d'ajustement du pH de la biomasse prétraitée ou du mélange de biomasses prétraitées avant ou pendant l'étape c) d'hydrolyse enzymatique, notamment un ajustement du pH entre 4 et 6. Quand on a donc affaire à un marc acide/neutre, il suffit généralement d'ajuster à la hausse le pH jusqu'à cette gamme de pH par appoint de base. Quand on a affaire à un mélange de marc dont du marc basique, on peut ajuster le pH uniquement en ajustant la quantité relative des deux types de marc dans le mélange, ou alors en prévoyant en outre un appoint de base/d'acide si nécessaire pour atteindre la gamme de pH voulue. Cet ajustement de pH vers des pH acides mais moins élevés que les pH rencontrés dans les prétraitements en conditions acides (où la biomasse peut « descendre « à des valeurs de pH de 2 à 3,5) est en effet généralement favorable à l'activité des enzymes de type cellulase utilisées dans l'hydrolyse enzymatique.

Avantageusement, sur un temps de production donné, la somme des durées des étapes de prétraitement b) de la biomasse préalablement mise dans des conditions acides ou à pH neutre dans un réacteur de prétraitement, pour produire un marc prétraité acide ou neutre, est supérieure, notamment au moins 2 à 5 fois, et de préférence entre 5 et 20 fois supérieure, à la somme des durées des étapes b') de prétraitement de la biomasse préalablement mise dans des conditions acide, neutre ou basique, avec éventuelle introduction suffisante dans le réacteur de prétraitement d'une solution aqueuse basique, pour produire un marc prétraité basique.

Selon une variante, le débit d'alimentation en biomasse du réacteur de prétraitement de l'étape de prétraitement b) est le même que le débit d'alimentation de l'étape b'). Selon une autre variante de l'invention, le débit d'alimentation en biomasse de l'étape b) de prétraitement est supérieur au débit d'alimentation en biomasse de l'étape b'). Ainsi, sur une durée donnée, le ratio de la somme des quantités de biomasse traitées selon l'étape b) sur les quantités de biomasses traitées selon l'étape b') est de préférence supérieur au ratio de leurs temps de production respectifs.

Selon l'invention, on préfère donc avoir des prétraitements en conditions basiques moins longs que ceux en conditions acides/neutre, à la fois opération par opération et sur toute la durée de production (que l'on comprend comme la durée entre le démarrage de l'installation et son arrêt, notamment pour des raisons de maintenance) : le prétraitement en conditions acides/neutre reste de préférence largement majoritaire en durée, celui en conditions basiques n'étant opéré qu'essentiellement que pour des raisons de nettoyage du réacteur de prétraitement et est limité au temps juste nécessaire à cela.

Avantageusement, on peut réaliser une séparation entre biomasse et phase aqueuse sous forme liquide ou vapeur en sortie du réacteur de prétraitement par un dispositif de séparation ou plusieurs dispositifs de séparation en parallèle, notamment deux dispositifs de séparation, fonctionnant en alternance.

Selon l'invention, optionnellement, chaque étape b') de prétraitement de la biomasse préalablement mise dans des conditions acide, neutre ou basique, avec introduction suffisante, le cas échéant, dans le réacteur de prétraitement d'une solution aqueuse basique, pour produire un marc prétraité basique, est suivie d'au moins un rinçage du dispositif de séparation ou d'un des dispositifs de séparation par une solution aqueuse (E ), notamment entre 1 et 10 rinçages successifs.

Selon un mode de réalisation particulier, on peut utiliser plusieurs dispositifs de séparation en parallèle fonctionnant en alternance, en sortie du réacteur de prétraitement, et, dans ce cas, on peut réaliser le rinçage d'un des dispositifs de séparation en discontinu pendant que le ou les autres dispositifs de séparation continuent de fonctionner.

Avantageusement selon l'invention, lors de l'étape c) d'hydrolyse enzymatique dans le réacteur d'hydrolyse, le mélange du marc prétraité acide MA ou neutre issu de l'étape b) avec le marc prétraité basique MB issu de l'étape b') se fait dans un ratio MA/MB en poids d'au moins 80/20 exprimé en poids brut notamment une teneur en marc MA supérieure à 85%poids, et de préférence 90%poids ou 95%poids, exprimé en poids brut du mélange des marcs et/ou une teneur en marc basique d'au moins 3%, notamment d'au moins 5% ou 8% ou 10%, notamment compris entre 5 et 15%.

On peut réaliser l'hydrolyse enzymatique de différentes façons : cette étape peut être réalisée en batch (en discontinu), en batch alimenté (ou « fed-batch » selon la terminologie anglo-saxonne) ou en continu. Par exemple, l'hydrolyse enzymatique peut être réalisée avec une première phase batch dans laquelle est introduit le marc basique MB en mélange avec du marc acide/neutre MA dans le réacteur d'hydrolyse, cette introduction étant suivie d'une mise au pH approprié par ajustement avec une solution de neutralisation, suivie d'une introduction des enzymes. Cette phase batch initiale peut être poursuivie par une phase de fed-batch de marc, qui peut être réalisée par alimentation avec du marc acide/neutre MA uniquement, ou par un mélange de marc acide/neutre MA avec du marc basique MB. A la fin de l'alimentation fed-batch, l'hydrolyse peut être poursuivie en mode batch pendant une durée déterminée, avant vidange de l'ensemble du réacteur en fin de réaction.

De préférence, l'étape de prétraitement b) est précédée d'une étape a) d'imprégnation dans un réacteur d'imprégnation de la biomasse par une solution aqueuse acide ou neutre.

L'invention a également pour objet une installation de traitement de biomasse lignocellulosique (notamment pour mettre en œuvre le procédé décrit précédemment), et qui comprend, d'amont en aval, :
- un réacteur d'imprégnation de biomasse en connexion fluidique avec une cuve de préparation de solution aqueuse acide,
- un réacteur de prétraitement de la biomasse imprégnée en connexion fluidique avec une cuve de préparation d'une solution aqueuse basique,
- un dispositif de séparation de la biomasse prétraitée en aval du réacteur de prétraitement, qui est optionnellement associé à des moyens de rinçage par une solution aqueuse et qui est en connexion fluidique avec
- optionnellement une zone de stockage intermédiaire de biomasse imprégnée de solution aqueuse acide, et
- une autre zone de stockage intermédiaire de biomasse imprégnée de solution basique, et
- un réacteur d'hydrolyse enzymatique alimenté en biomasse prétraitée depuis la/l'une au moins desdites zones de stockage.

On comprend les termes « amont » et « aval » en fonction de la direction générale du cheminement de la biomasse dans l'installation.

L'installation peut aussi comprendre une zone d'ajustement du pH de la biomasse entre au moins la /une des zones de stockage et le réacteur d'hydrolyse enzymatique, notamment une zone d'ajustement du pH commune pour les biomasses issues des zones de stockage.

Avantageusement, selon une variante, cette zone d'ajustement du pH peut être une cuve assurant le mélange des biomasses issues de chacune des zones de stockage.

Selon une autre variante, l'ajustement de pH se fait dans le réacteur d'hydrolyse avant ou pendant l'étape d'hydrolyse enzymatique.

Le mélange des marcs peut se faire directement dans le réacteur d'hydrolyse enzymatique ou préalablement à leur introduction dans le réacteur.

Selon une variante, le marc acide/neutre et le marc basique sont introduits par le même système d'introduction dans le réacteur d'hydrolyse enzymatique, ce système pouvant aussi servir à l'introduction du mélange des marcs acide/neutre et basique.

Selon une autre variante, deux systèmes d'alimentation différents en marc sont prévus selon que l'alimentation est réalisée avec du marc acide/neutre ou avec du marc basique ou du mélange de marcs).

Sont données ci-après des précisions sur la façon d'opérer l'imprégnation éventuelle, le prétraitement et la séparation après prétraitement selon l'invention : (l'étape b') de prétraitement en conditions basiques pourra aussi être désignée sous l'expression étape de nettoyage du réacteur de prétraitement : elles correspondent à la même étape).

Avantageusement, on peut prévoir de chauffer la solution aqueuse basique avant son introduction dans le réacteur de prétraitement, notamment à une température d'au moins 40°C, notamment d'au moins 80°C. Il a été constaté que la solution basique était efficace plus rapidement si elle était ainsi préchauffée hors du réacteur.

Avantageusement encore, l'introduction de la solution basique dans le réacteur de prétraitement peut s'effectuer dans le réacteur de prétraitement dont le volume intérieur est à une température d'au moins 120°C, notamment d'au moins 140°C. Cette température interne du réacteur peut être celle à laquelle le prétraitement est réalisé de manière conventionnelle, notamment par cuisson. L'effet de la solution basique sur les résidus solides collés aux parois du réacteur est en effet amplifié quand la solution/le réacteur sont chauds.

Selon un mode de réalisation de l'invention, on introduit la biomasse dans le réacteur de prétraitement par un moyen d'amenée avec mise sous pression, du type vis convoyeuse conique, qui est lavé par une solution aqueuse, et on recycle cette solution de lavage pour préparer la solution aqueuse basique utilisée lors du nettoyage dudit réacteur. On diminue ainsi la consommation en eau, accrue, du procédé de prétraitement due à l'utilisation d'une solution aqueuse basique de nettoyage.

De préférence, la phase de nettoyage du réacteur de prétraitement avec la solution basique a une durée comprise entre 15 minutes et 8 heures, notamment entre 1 et 3 heures. Cette durée n'est donc pas très longue, et peut être modulée en fonction de la fréquence avec laquelle le réacteur est nettoyé.

Lors de la phase de nettoyage du réacteur de prétraitement, le débit de la solution aqueuse basique en entrée dudit réacteur est de préférence réglé de manière à ce que la teneur en matière sèche MS de la biomasse diminue significativement lors de son passage dans le réacteur, de par exemple une valeur de 30 à 60% MS, notamment de 50% MS, à une valeur de 15 à 25% MS, notamment de 25% MS. En effet, la solution aqueuse basique va avoir un premier rôle, qui est d'imprégner d'eau la biomasse entrante dans le réacteur jusqu'à la saturation de la biomasse en liquide.

Dans tout le présent texte, l'acronyme "MS" désigne le taux de matière sèche qui est mesurée selon la norme ASTM E1756 - 08(2015) « Standard Test Method for Determination of Total Solids in Biomass".

Ensuite, et c'est son second rôle, on règle de préférence la concentration en base (KOH par exemple) de la solution aqueuse basique de façon à augmenter le pH de la biomasse entrant dans le réacteur depuis une gamme de pH acide comprise entre 0,5 et 3, de préférence au voisinage de 3, vers une gamme de pH basique comprise entre 8 et 14, de préférence au voisinage de 13. (On est alors dans le cas de figure d'un prétraitement d'une biomasse pré-imprégnée d'une liqueur acide, et qu'il faut donc basculer à un pH basique pour effectuer le nettoyage selon l'invention.)

En jouant sur le débit et la concentration en base de la solution, on peut saturer en eau et remonter le pH de la biomasse, les quantités précises de solution basique consommée lors d'un nettoyage dépendant naturellement de la taille du réacteur et des caractéristiques de la biomasse entrant dans le réacteur.

De préférence, le taux de remplissage du réacteur par la biomasse pendant le traitement est compris entre 20 et 80% ou 90%. Pendant la phase de nettoyage, ce taux de remplissage peut être maintenu dans cette fourchette, et même, de préférence, (un peu) augmenté (par exemple en diminuant la vitesse de rotation de la vis ou d'au moins une des vis internes de convoyage du réacteur quand il en possède), ce qui tend à améliorer la qualité du nettoyage La phase de nettoyage en continu du réacteur de prétraitement est réalisée selon une fréquence donnée et/ou quand il y a dépassement d'une valeur-seuil d'une caractéristique physico-chimique ou rhéologique du milieu réactionnel dans ledit réacteur. Cette caractéristique peut être mesurée ou évaluée de façon directe ou indirecte. Il peut s'agir, par exemple, d'un seuil de puissance du moteur utilisé pour faire tourner une vis de convoyage dans le réacteur, pour faire tourner des moyens d'agitation dans le réacteur ou toute autre pièce mobile dans celui-ci.

Selon un mode de réalisation, on peut changer une des caractéristiques physiques, chimiques ou rhéologiques ou la nature de la biomasse alimentant le réacteur de prétraitement pendant au moins une partie de sa phase de nettoyage. En effet, il peut être intéressant, le temps du nettoyage, d'alimenter le réacteur de prétraitement avec une biomasse avec un pouvoir abrasif supérieur à la biomasse utilisée pendant le reste du temps de production. On peut par exemple, ainsi, remplacer de la biomasse de type paille pendant la production par une biomasse de type peuplier pendant le nettoyage.

Avantageusement, le prétraitement est selon l'invention une cuisson avec explosion à la vapeur. On peut alors épuiser thermiquement la chaleur de la vapeur récupérée, notamment dans un dispositif de séparation de type cyclone en sortie du réacteur de prétraitement, pour chauffer la ou une des solutions aqueuses utilisées dans ledit procédé.

Le prétraitement peut s'effectuer par cuisson, avec ou sans explosion à la vapeur.

Selon un mode de réalisation préféré, on imprègne la biomasse avec une solution aqueuse acide dans un réacteur d'imprégnation, avant son introduction dans le réacteur de prétraitement. Les deux réacteurs peuvent être montés en série et fonctionner en continu. Le fait que, temporairement, la biomasse acidifiée soit mise en contact avec une solution basique dans le réacteur de prétraitement n'a pas affecté significativement la qualité ou le rendement de la production de biomasse prétraitée : même si la biomasse prétraitée pendant le nettoyage peut présenter des différences d'avec la biomasse prétraitée hors nettoyage, notamment des valeurs de pH qui augmentent puis baissent à nouveau progressivement, elle reste exploitable, dans la mesure où, comme expliqué plus haut, on la combine dans des proportions appropriées avec de la biomasse prétraitée en conditions acide/neutre avant hydrolyse enzymatique.

Selon un mode de réalisation, pendant au moins une partie de la phase de nettoyage en continu du réacteur de prétraitement, on diminue ou on supprime la teneur en acide de la solution aqueuse acide mise en contact avec la biomasse lors de son imprégnation préalable dans le réacteur d'imprégnation. On peut ainsi réduire la quantité de base nécessaire à la préparation de la solution basique de nettoyage, puisque la quantité d'acide contenue dans la biomasse à neutraliser sera ainsi moins élevée.

Selon un autre mode de réalisation, pendant au moins une partie de la phase de nettoyage en continu du réacteur de prétraitement, on remplace la solution aqueuse acide mise en contact avec la biomasse, lors de son imprégnation préalable dans le réacteur d'imprégnation, par une solution aqueuse basique, notamment la même que celle qui est injectée pendant ladite phase dans le réacteur de prétraitement : on peut ainsi encore réduire la consommation de base nécessaire au nettoyage par rapport au mode de réalisation précédent. On peut également la remplacer par une solution aqueuse de pH neutre, toujours pour la même raison.

Selon une variante, on utilise plusieurs, notamment deux, réacteurs d'imprégnation en parallèle pour imprégner la biomasse avec une solution aqueuse avant son introduction dans le réacteur de prétraitement : un premier réacteur d'imprégnation est alimenté en solution aqueuse acide, et un deuxième réacteur d'imprégnation est alimenté en solution aqueuse basique ou en solution aqueuse de pH neutre, les deux réacteurs fonctionnant en alternance, le deuxième réacteur étant opérationnel pendant au moins une partie de la phase de nettoyage du réacteur de prétraitement. Cette variante permet de mettre en œuvre les modes de réalisation précédents, en modifiant le type d'imprégnation de la biomasse avant son prétraitement quand le réacteur de prétraitement passe en mode nettoyage, en basculant l'arrivée de biomasse à imprégner d'un réacteur d'imprégnation à l'autre.

De préférence, on peut réaliser une séparation entre biomasse et phase aqueuse sous forme liquide ou vapeur en sortie du réacteur de prétraitement, par un dispositif de séparation ou plusieurs dispositifs de séparation en parallèle, notamment deux dispositifs de séparation, fonctionnant en alternance. Il peut s'agir d'équipements de type cyclones. Comme précédemment pour les deux réacteurs d'imprégnation en parallèle, utiliser plusieurs dispositifs de séparation en parallèle permet de réduire la période de transition entre le mode production et le mode production + nettoyage du réacteur de prétraitement : avec deux dispositifs en parallèle, un des deux dispositifs peut être dédié à la séparation de la biomasse prétraitée « basique » (celle prétraitée pendant le nettoyage), en ne séparant que la biomasse prétraitée pendant le nettoyage, et l'autre dispositif est alors dédié à la séparation conventionnelle de la biomasse prétraitée avec imprégnation acide ou neutre (celle prétraitée hors période de nettoyage).

L'installation selon l'invention peut comprendre deux réacteurs d'imprégnation et/ou deux dispositifs de séparation et/ou deux réacteurs de prétraitement fonctionnant en alternance selon que le prétraitement est opéré sur de la biomasse préalablement mise dans des conditions acides ou à pH neutre pour produire un marc prétraité acide ou neutre, selon l'étape b), ou sur de la biomasse préalablement mise dans des conditions acide, neutre ou basique, avec introduction suffisante dans le réacteur de prétraitement d'une solution aqueuse basique (EB), pour produire un marc prétraité basique selon l'étape b').

La mise en œuvre de l'invention proposée ajoute donc simplement à une installation existante, dans son mode de réalisation le plus simple :
- éventuellement une cuve de préparation de la solution basique, qui pourra être alimentée en eau, et en base concentrée (KOH, NaOH par exemple, ou toute autre base minérale ou organique), et éventuellement en eau/solution basique recyclée. Cette cuve est équipée de moyens connus pour, notamment, maintenir constant le pH de la solution, par ajout de base (KOH) et/ou d'eau. La cuve peut également être munie de moyens chauffants pour amener/maintenir la solution basique à la température voulue avant introduction dans le réacteur. Les moyens chauffants peuvent, alternativement ou cumulativement, être prévus sur les moyens de connexion fluidique, type conduites, amenant la solution basique depuis la cuve vers le réacteur,
- des moyens pour réaliser le mélange entre le marc prétraité en conditions acide/neutre et le marc prétraité en conditions basiques (une zone « tampon » de stockage intermédiaire de marc basique, éventuellement une autre pour le stockage de marc acide/neutre ; voire une troisième pour réaliser le mélange et un ajustement de pH avant envoi dans le réacteur d'hydrolyse). La ou les zones tampon peuvent se présenter sous forme de cuves ou sous forme de tout autre moyen connu de stockage intermédiaire, comme une trémie par exemple.

L'invention a également pour objet l'utilisation du procédé ou de l'installation décrits plus haut pour le traitement de biomasses du type bois, paille, résidus agricoles, et toutes cultures énergétiques dédiées, notamment des plantes annuelles ou pluriannuelles telles que le miscanthus, en vue de produire des sucres, des biocarburants ou des molécules bio-sourcées.

L'invention a également pour objet un procédé de traitement de biomasse lignocellulosique comprenant les étapes suivantes:
- la préparation d'une liqueur d'imprégnation contenant un catalyseur, notamment acide,
- l'introduction de la biomasse dans un réacteur d'imprégnation pour être imprégnée par la liqueur d'imprégnation,
- le transfert de la biomasse imprégnée dans un réacteur de prétraitement pour y subir un prétraitement par cuisson,
- l'hydrolyse enzymatique de la biomasse prétraitée,
- la fermentation alcoolique du moût d'hydrolyse enzymatique obtenu, tel que le procédé est réalisé en continu sur tout ou partie desdites étapes, et tel qu'on opère un nettoyage du réacteur d'imprégnation sans interrompre sa production en biomasse prétraitée.

L'installation en question peut utiliser deux réacteurs d'imprégnation et/ou deux dispositifs de séparation et/ou deux réacteurs de prétraitement, fonctionnant en alternance selon que le réacteur de prétraitement est en phase de nettoyage ou non.

L'invention a également pour objet toute installation de mise en œuvre du procédé de traitement de biomasse mentionné plus haut, telle que cette installation comprend, successivement :
- un réacteur d'imprégnation alimenté en solution d'imprégnation par une cuve de préparation de ladite solution, et en biomasse,
- un réacteur de prétraitement de la biomasse imprégnée que l'on peut alimenter en solution aqueuse basique par une cuve de préparation de ladite solution,
- un réacteur d'hydrolyse enzymatique, et
- un réacteur de fermentation alcoolique qui peut être le même que le réacteur d'hydrolyse, l'ensemble des réacteurs étant montés en série, ou au moins deux d'entre eux.

A noter que l'hydrolyse et la fermentation peuvent aussi être réalisées en même temps dans le même réacteur.

### Liste des figures

La figure 1 représente le schéma bloc d'un mode de réalisation d'un procédé de traitement de biomasse lignocellulosique selon l'invention.
La figure 2 représente une portion de l'installation apte à mettre en œuvre le procédé selon la figure 1.
La figure 3 représente l'installation mettant en œuvre une première variante de la première séquence T1 du procédé selon l'invention représenté à la figure 1.
La figure 4 représente l'installation mettant en œuvre une deuxième variante de la première séquence T1' du procédé selon l'invention représenté à la figure 1.
La figure 5 représente l'installation mettant en œuvre une première variante de la deuxième séquence T2 du procédé selon l'invention représenté à la figure 1.
La figure 6 représente l'installation mettant en œuvre une deuxième variante de la deuxième séquence T2' du procédé selon l'invention représenté à la figure 1.
La figure 7 représente un graphe de suivi de production d'éthanol selon un exemple non conforme à l'invention.
La figure 8 représente un graphe de suivi de production d'éthanol selon un exemple conforme à l'invention.
La figure 9 représente un graphe de suivi de production d'éthanol selon un exemple conforme à l'invention.
La figure 10 représente un graphe de suivi de production d'éthanol selon un exemple conforme à l'invention.
La figure 11 représente un graphe de suivi de production d'éthanol selon un exemple conforme à l'invention.

### Description des modes de réalisation

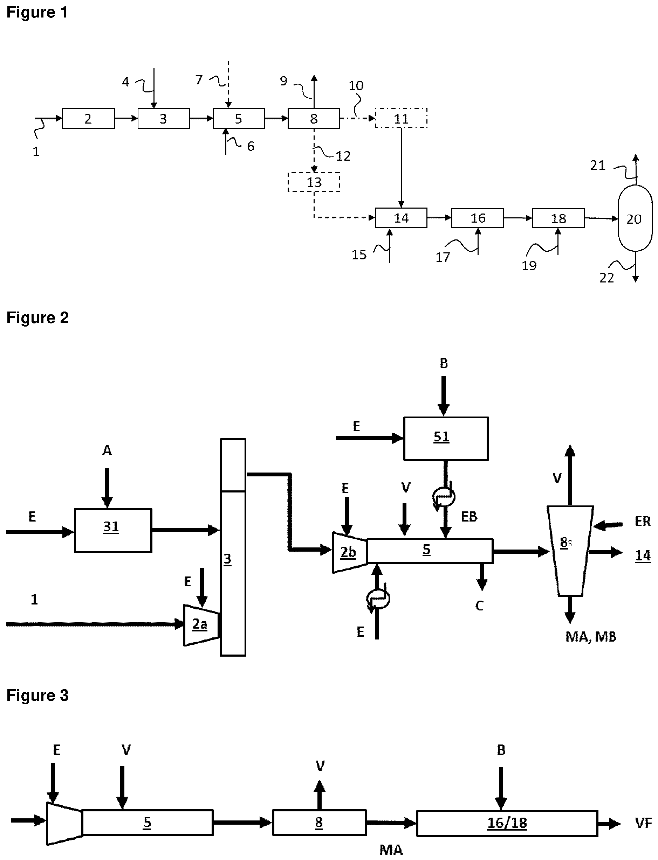

Les figures 1 à 5 sont très schématiques, les mêmes références correspondent aux mêmes composants d'une figure à l'autre. La figure 1 est un schéma bloc. Dans les figures suivantes, les réacteurs et autres équipements sont représentés dans la position spatiale qu'ils occupent sensiblement en position opérationnelle, sans les détails ou les équipements de moindre importance au vu de l'invention.

Le procédé de traitement de biomasse lignocellulosique incluant le nettoyage en continu d'un réacteur de prétraitement de biomasse en conditions basiques en alternance avec un prétraitement conventionnel en conditions acide ou neutre est ici illustré dans le cadre d'un procédé de traitement de biomasse destiné à produire des alcools, notamment du biocarburant du type bioéthanol. Les grandes étapes de ce procédé sont décrites à la figure 1, décrit rapidement ci-dessous. Un mode de réalisation de ce procédé est décrit plus en détails, par exemple, dans le brevet WO 2018/015227 auquel on se rapportera au besoin.

Il est à noter que le procédé de l'invention peut s'appliquer de la même manière à tout prétraitement suivi d'une hydrolyse enzymatique de biomasse, et à tout réacteur qui est destiné à traiter une biomasse lignocellulosique.

Le procédé de traitement de biomasse ici pris en exemple et représenté à la figure 1 comporte schématiquement les grandes étapes suivantes : une première étape d'imprégnation de la biomasse dans un réacteur vertical, suivie d'une étape de prétraitement de la biomasse une fois imprégnée dans un réacteur d'explosion à la vapeur 4 horizontal, puis une étape d'hydrolyse enzymatique puis d'une fermentation alcoolique (hydrolyse et fermentation pouvant se faire simultanément dans le même réacteurs ou l'une après l'autre), puis fractionnement par distillation. C'est dans cette succession d'étapes que l'invention s'insère, comme représenté à la figure 1 décrite ci-après.

Les références de la figure 1 correspondent aux équipements et composés suivants, d'amont en aval, dans l'installation de traitement de la biomasse :
1 : biomasse native (c'est-à-dire avant tout traitement)
2 : conditionnement de la biomasse native (broyage, épierrage, dépoussiérage éventuel, etc...)
3 : zone de contactage de la biomasse conditionnée avec une liqueur (phase aqueuse) acide (imprégnation)
4 : liqueur acide (H₂SO₄ en phase aqueuse)
5 : réacteur de prétraitement par cuisson - fonctionnement en continu
6 : injection de vapeur - fonctionnement en continu
7 : injection de solution aqueuse de base - fonctionne durant la séquence T2 uniquement (détaillée plus loin)
8 : équipement de séparation type cyclone - fonctionne en continu
9 : vapeurs séparées du substrat biomasse prétraité - évacuées en continu
10 : sortie du marc produit en conditions acides - fonctionne durant la séquence T1 uniquement (détaillée plus loin)
11 : zone tampon de marc acide, alimentée uniquement pendant la séquence T1 et soutirée pendant T1 et pendant T2
12 : sortie du marc produit en conditions basiques - fonctionne durant la séquence T2 uniquement
13 : zone tampon de marc basique, alimentée uniquement pendant la séquence T2 et soutirée au moins en partie pendant T1
14 : zone de neutralisation du marc pour atteindre un pH entre 4 et 6
15 : solution de neutralisation
16 : réacteur d'hydrolyse enzymatique
17 : cocktail d'enzymes et autres nutriments nécessaires + réajustement de Ph
18 : zone de fermentation éthanolique (séparée ou concomitante à l'hydrolyse) - sortie CO₂ non représentée
19 : levures pour fermentation et autres nutriments nécessaires + réajustement de pH
20 : dispositif de récupération de l'éthanol, par exemple une distillation
21 : produit éthanol
22 : résidus (solides et liquides, en mélanges ou séparés selon l'agencement de 20)

Reprenons la figure 1 en décrivant tout d'abord les étapes conventionnelles du procédé : La biomasse native 1 est d'abord conditionnée dans l'étape 2, notamment pour lui donner une taille de particules donnée (broyage), pour retirer les pierres et autres composants qui ne font pas partie de la biomasse elle-même. Puis elle est imprégnée d'une solution aqueuse acide 4 dans un réacteur d'imprégnation 3. La biomasse imprégnée acide est ensuite amenée dans un réacteur de prétraitement 5 à haute température avec injection de vapeur 6 pour y subir une cuisson par explosion à la vapeur. Une phase vapeur 9 est ensuite séparée de la biomasse prétraitée également appelé marc, ici un marc acide MA donc, dans un équipement de séparation 8 de type cyclone. Ensuite, ce marc acide est amené dans une zone de neutralisation 14 pour ajuster le pH à une valeur un peu supérieure, pour passer d'un pH de l'ordre de 2 à 3,5 à un pH de l'ordre de 4 à 6 par ajout d'un agent neutralisant 15, puis ce marc neutralisé MN subit une hydrolyse enzymatique dans un réacteur ad hoc 16 alimenté avec un cocktail enzymatique 17 approprié, puis une fermentation éthanolique 18 (qui peut être concomitante à l'hydrolyse), dans le même réacteur ou dans un réacteur distinct, avec apport des levures de fermentation 19 appropriées. Enfin, on prévoit de récupérer l'alcool 21 en le séparant des résidus solides et liquides 22 dans un dispositif 20, qui peut être une colonne de distillation.

Décrivons maintenant ce qu'apporte l'invention à cette succession d'étapes conventionnelles:
L'invention propose un fonctionnement en deux séquences T1 et T2 qui s'alternent.
- La séquence T1 est la séquence conventionnelle, illustrée également par la figure 2, avec, en sortie du réacteur de prétraitement 4, un marc acide 10, mais ici ce marc MA, au lieu d'être envoyé directement en zone de neutralisation 14 après séparation dans le dispositif de séparation 8, passe par une zone tampon 11 de marc acide 10, une trémie par exemple, qui n'est alimentée en marc que pendant la séquence T1,
- La séquence T2 est la séquence où le réacteur de prétraitement est alimenté en solution aqueuse basique 7, notamment par une cuve de préparation connectée au réacteur de prétraitement 5, dont on soutire alors un marc basique 12, qui vient alimenter une zone tampon 13 de marc basique 12 (ou MB).
- La zone de neutralisation 14 est alimentée indépendamment des séquences T1 et T2. Pendant une séquence T3, elle est alimentée uniquement par du marc acide 10 provenant de la zone tampon 11 et/ou directement par le marc en sortie de réacteur 10 lorsque celui-ci est fonctionnement selon la séquence T1. Pendant une séquence T4, elle est alimentée par un mélange de marc acide 10 (ou MA) provenant de la zone 11 et de marc basique 12 (ou MB) provenant de la zone 13, selon une proportion définie. La neutralisation à réaliser dans la zone 14 est alors moins importante que pendant la séquence T3, puisque le mélange de mars est à un pH supérieur à celui du marc acide seul, on peut même éventuellement ne plus avoir besoin de faire de neutralisation par appoint de base à ce stade. Les étapes suivantes restent inchangées. Alternativement, si le ratio entre les deux types de marc dans le mélange est choisi en conséquence, on peut « by-passer » la zone de neutralisation 14 pendant la séquence T4 et injecter directement le mélange de marcs ou chacun des mars séparément provenant de leurs zones tampon respectives dans le réacteur 16 d'hydrolyse enzymatique.

Dans une variante de mise en œuvre, la zone de neutralisation 14 peut se situer dans le réacteur d'hydrolyse enzymatique 16. Celui-ci reçoit alors les marcs et la solution de neutralisation 15 en complément de la solution enzymatique 17.

L'installation apte à mettre en œuvre l'invention selon le procédé de la figure 1, jusqu'à l'étape de séparation avec le dispositif 8, est représentée à la figure 2 : Ces étapes du procédé sont réalisées en continu et sont détaillées ci-dessous à l'aide de la description des équipements utilisés pour le mettre en œuvre:
- Une cuve de préparation 31 d'une liqueur d'imprégnation contenant un catalyseur chimique est prévue, qui est constituée à partir d'eau E et de catalyseur A qui viennent l'alimenter, le catalyseur est en l'occurrence un acide fort de type acide sulfurique concentré en phase aqueuse, cette cuve permettant d'alimenter le réacteur d'imprégnation 3 en un mélange d'eau E et de catalyseur chimique A,
- Une vis conique 2a (appelée aussi sous la terminologie anglo-saxonne « plug screw » ou « sealing screw ») d'alimentation en biomasse fraîche 1 (ici de la paille de blé) dans le réacteur d'imprégnation 3,
- Une alimentation en liqueur d'imprégnation du réacteur reliant la cuve de préparation de liqueur 31 et le réacteur d'imprégnation 3,
- Un réacteur d'imprégnation 3 équipé de deux vis de transport ascendant (non représentées) permettant à la biomasse de passer de la zone d'imprégnation en partie inférieure du réacteur à la zone d'égouttage en partie supérieure du réacteur, et d'amener la biomasse imprégnée et égouttée en sortie de réacteur située en haut du réacteur. Cette biomasse imprégnée et égouttée est ensuite envoyée au prétraitement par une alimentation débouchant dans une deuxième vis conique 2b.
- Cette deuxième vis conique 2b alimente en biomasse imprégnée le réacteur de prétraitement 5,
- Le réacteur de prétraitement 5 traite la biomasse imprégnée par explosion à la vapeur,
- Un circuit d'eau de lavage des vis coniques 2a,2b du réacteur d'imprégnation 3 et du réacteur de prétraitement 5, représentés symboliquement à la figure 1 par des arrivées d'eau E au niveau desdites vis, est prévu
- Un moyen de séparation de la vapeur 8 est alimentée par le réacteur 5 en biomasse ayant subi la cuisson par explosion à la vapeur, de type cyclone par exemple, avec en sortie haute de la vapeur V et en sortie basse la biomasse prétraité/explosée, appelée aussi moût acide (ou marc acide) MA.

Ce marc MA présente à ce stade une accessibilité de la cellulose aux enzymes suffisante pour être traité par hydrolyse enzymatique pour la production de sucres 2G. Les conditions de l'hydrolyse enzymatique et de la fermentation consécutive ou simultanée qui suivent cette séparation (non représentées à la figure 1) sont adaptées aux produits souhaités et sont connues de l'homme du métier.

L'utilisation de la technique de prétraitement décrite plus haut conduit au dépôt de différents types de biomasse (de la paille de blé ici, mais aussi miscanthus, peuplier, etc.), qui s'accumulent/adhérent à la surface de la vis 2b et du réacteur de prétraitement 5. Ces dépôts subissent une cuisson sur des temps plus longs que le temps de séjour normal de la biomasse dans le réacteur, et se transforment en un résidu, qu'on peut appeler ici « coke ». Ce « coke » peut créer divers problèmes opératoires, comme des bouchages de l'orifice de sortie du réacteur 4, une augmentation des frottements de la vis 2b sur la paroi de l'enceinte dans laquelle elle est logée, et il peut en résulter une réduction des performances de l'unité de prétraitement dans son ensemble telle que représentée à la figure 1.

La définition de la composition du « coke » s'est avérée délicate, car il s'agit d'un résidu dont l'aspect et la composition évoluent au fur et à mesure du temps : au début d'un cycle de production, la matière qui se dépose est de la biomasse, elle a donc essentiellement les mêmes caractéristiques que la biomasse qui continue son parcours au travers du réacteur 4 et vers les étapes aval. Le dépôt qui se constitue par adhérence à la paroi interne du réacteur 4 va rester un temps beaucoup plus long dans les conditions de cuisson (température notamment) que souhaité. L'effet de la température affecte la composition et la morphologie du résidu, qui va évoluer vers un résidu de plus en plus « cuit ». Plus le résidu est « cuit », plus il est compact et plus il adhère aux parois du réacteur.

Ces dépôts de « coke » sont cumulatifs : plus le temps d'opération de l'outil en continu est long, plus la quantité de coke déposée sera importante, et plus les « couches » de coke proches de la paroi vont évoluer vers un solide très dur. Ces dépôts provoquent donc un phénomène d'encrassement, en augmentant l'épaisseur des parois et en diminuant le volume utile du réacteur. Selon la configuration du réacteur de cuisson, et notamment le type d'interne en place, on peut constater une gêne à la rotation de certains éléments comme la vis de convoyage de la biomasse en cours de cuisson. Cette gêne s'observe notamment par une montée de la puissance du moteur faisant tourner la vis.

Tout au long de la production, il peut aussi arriver qu'une partie de ce résidu, plus ou moins durcie, se décroche de la paroi du réacteur, sous l'effet, par exemple, de la rotation de la vis ou du passage de la biomasse au travers du réacteur: ainsi, des particules de densité bien supérieure au lit de biomasse en cours de cuisson peuvent être amenées à se détacher et à être entraînées vers l'orifice de sortie du réacteur, ce qui peut générer des bouchages ou des problèmes opératoires en aval. Malgré ces décrochages, on constate que les dépôts continuent d'augmenter au cours du temps pendant un cycle de production donné.

Après arrêt, refroidissement et ouverture du réacteur 4 de cuisson, on a pu constater que le coke se présente sous deux formes : une forme dure au contact direct des parois internes du réacteur et une forme plus friable qui recouvre le coke dur. La différence entre ces deux cokes se trouve dans leurs compositions élémentaires, comme indiqué dans le tableau 1 ci-dessous.

| | Coke friable | Coke dur |
|---|---|---|
| Teneur en carbone (%) | 44,57 | 65,07 |
| Teneur en hydrogène (%) | 5,85 | 4,67 |
| Teneur en oxygène (%) | 34,63 | 24,58 |

On constate que le pourcentage en carbone contenu dans le coke dur est plus élevé que celui dans le coke friable, tandis qu'on note une tendance inverse pour la teneur en oxygène, et des valeurs similaires pour la teneur en hydrogène. Il en ressort que le coke friable est en quelque sorte le précurseur du coke dense.

L'invention consiste à poursuivre le fonctionnement des deux réacteurs d'imprégnation 3 et de prétraitement 5, tout en procédant au nettoyage chimique du réacteur 5 afin d'extraire ce coke C et/ou d'en ralentir la formation, sans « perdre » le mout produit lors de ce nettoyage en conditions basiques.

Ce nettoyage ne nécessite pas l'ouverture du réacteur et le nettoyage mécanique de l'intérieur du réacteur comme c'était le cas précédemment. Ce nettoyage selon l'invention, détaillé plus bas, est donc plus rapide, plus économique et plus sécuritaire, puisqu'il permet de limiter les risques opératoires liés au montage et démontage de l'unité, et, surtout, puisqu'il permet de ne pas stopper la production.

Un exemple de mise en œuvre du procédé selon l'invention nécessite les équipements supplémentaires suivants, par rapport à ceux déjà décrits, au vu de la figure 1 :
- Une cuve de préparation 51 d'une liqueur de nettoyage/liqueur basique EB contenant une base. Cette cuve 51 permet d'alimenter le réacteur de prétraitement 5 en solution basique à une certaine concentration. Elle est alimentée en eau E et en base B (par exemple d'une base B sous forme d'une solution aqueuse concentrée en KOH), dont l'appoint est ajusté pour obtenir une liqueur à la concentration en base, au pH voulus.
- Une alimentation en liqueur de nettoyage du réacteur 5 reliant la cuve de préparation de liqueur de nettoyage/liqueur basique 51 et le réacteur de prétraitement 5 à nettoyer, en la préchauffant le cas échéant par des équipements ad hoc (résistances chauffantes entourant les conduites par exemple), avec des équipements adaptés pour injecter la liqueur de nettoyage dans le réacteur 5 sous pression,
- Une arrivée d'eau optionnelle de rinçage ER pour le cyclone 8
- Sont également prévues, mais non représentées, une ou des cuves de stockage temporaire ou trémies ou autres moyens de stockage temporaires, les « zones tampon » mentionnées plus haut aptes à récupérer, dans une des cuves, le marc acide MA conventionnel, et dans l'autre cuve, le marc basique MB obtenu pendant le nettoyage en conditions basiques du réacteur de prétraitement 5.

Le déroulement d'un exemple de réalisation du procédé de nettoyage/séquence T1 selon l'invention comprend deux sous-séquences consécutives :
- sous-séquence 1 : Injection de la liqueur basique EB chauffée préalablement dans le réacteur 5 en cours d'alimentation du réacteur avec la biomasse acide.

Les conditions de l'injection sont les suivantes :
- la solution basique EB est une solution aqueuse de KOH, avec une concentration en KOH de 1 à 50% poids de KOH, de préférence de 5 à 12% poids de KOH , par rapport à l'eau
- le débit de la solution EB dans le réacteur est compris entre 100 et 500 kg/h, notamment d'environ 300 kg/h
- le taux de remplissage par la solution EB du réacteur 4 est de 20 à 50%, notamment d'environ 30%
- la température à laquelle la solution EB est injectée dans le réacteur 4 est comprise entre 80°C et 200°C, notamment d'environ 130°C
- la température du réacteur 4 est comprise entre 150 et 220°C, notamment d'environ 200°C
- la durée de cette séquence est comprise entre 15 minutes et 8 heures, elle est notamment de 2 heures
- le temps de séjour de la solution EB dans le réacteur 4 est compris entre 5 et 15 minutes, et notamment d'environ 10 minutes.
- sous-séquence 2 (optionnelle) : Nettoyage du cyclone 8 par la réalisation d'un rinçage à l'eau ER pour terminer le nettoyage. On peut parler de « flush d'eau », dans la mesure où le rinçage consiste, dans cet exemple de réalisation, à projeter sous pression de l'eau dans le cyclone, eau qui est ensuite rapidement évacuée.

Les conditions opératoires de cette séquence, si elle est réalisée, sont les suivantes :
- nombre de rinçages : de 1 à 10, par exemple égal à 2
- température de l'eau de rinçage : 20°C à 80°C, par exemple de 20°C (donc soit une température à l'ambiante ou proche de l'ambiante, soit une température plus élevée nécessitant un préchauffage de l'eau de rinçage ER)

En phase de nettoyage = séquence T2, on obtient en sortie du cyclone 8 un mout qui n'est plus le mout acide MA conventionnel, mais un mout basique MB.

La fréquence de la procédure de nettoyage/de la séquence 2 peut varier largement en fonction du type et de la taille du réacteur de prétraitement 5, du type de biomasse traitée... Par exemple, le nettoyage peut être déclenché quand le couple d'une des vis de transport internes au réacteur augmente de plus de 15% par rapport au couple observé en début de production. Il peut aussi être déclenché au bout d'une période donnée, qui peut aller de 2 heures à 4 mois de production.

La figure 3 décrit un mode de fonctionnement du procédé selon la séquence T1, depuis le réacteur de prétraitement jusqu'à la fermentation éthanolique : après la séparation par le cyclone 8 (ou tout autre dispositif de séparation liquide/gaz), le marc acide obtenu MA est conduit à l'hydrolyse enzymatique, avec ajustement du pH par appoint de base B directement dans le réacteur d'hydrolyse, de façon à obtenir après fermentation un vin de fermentation VF.

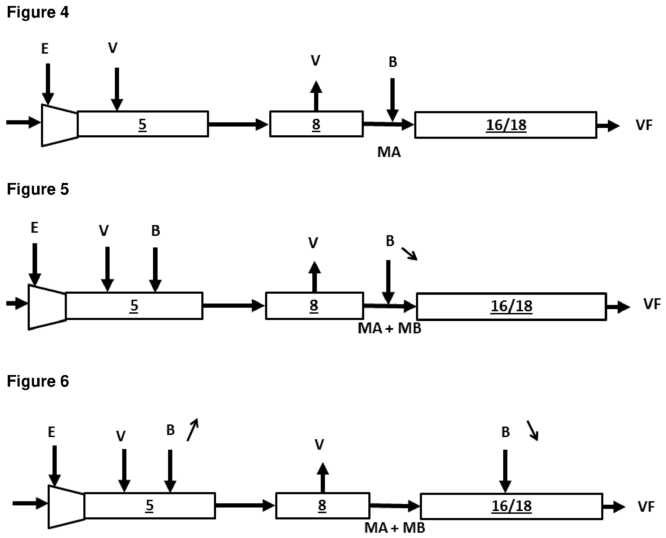

La figure 4 est une variante du procédé de la figure 3 : la différence est qu'ici on procède à l'ajustement du pH du marc acide MA avant introduction dans le réacteur d'hydrolyse, notamment par passage dans une zone/une cuve de neutralisation avec appoint de base, ou par introduction d'un appoint de base dans la conduite de transfert depuis le cyclone vers le réacteur d'hydrolyse.

La figure 5 décrit un mode de fonctionnement du procédé selon la séquence T2, depuis le réacteur de prétraitement jusqu'à la fermentation éthanolique : après la séparation par le cyclone 8 (ou tout autre dispositif de séparation liquide/gaz), le marc acide obtenu MB est ajouté à du marc acide MA stocké temporairement pour faire un mélange MA + MB qui est conduit à l'hydrolyse enzymatique, avec ajustement du pH par appoint de base B directement dans le réacteur d'hydrolyse, de façon à obtenir après fermentation un vin de fermentation VF. Alternativement, comme montré à la figure 6, l'appointé de base peut se faire en amont du réacteur d'hydrolyse. Dans les deux cas, l'appoint en base sera inférieur à l'appoint en base nécessaire pendant la séquence T1, puisque le mélange de marc MA + MB est globalement moins acide que le marc acide MA, selon la proportion de marc basique dans le mélange.

Différentes variantes peuvent être apportées à l'exemple de procédé de nettoyage/de séquence T2 décrit plus haut, tout en restant dans le cadre de l'invention, dont certaines sont détaillées ci-dessous (certaines au moins de ces variantes peuvent être alternatives ou cumulatives) :
A-En cours de nettoyage, la concentration en acide A de la cuve de préparation 31 de la liqueur d'imprégnation peut être réduite jusqu'à une concentration nulle éventuellement, soit, in fine, une imprégnation qui ne se fait plus qu'avec de l'eau.
B- L'eau de lavage E de la vis 2b amenant la biomasse imprégnée dans le réacteur 4 peut être recyclée dans la cuve de préparation 51 de la solution basique EB pendant le nettoyage, ce qui permet de réduire la consommation en eau supplémentaire due au nettoyage.
C- On peut réaliser une imprégnation de la biomasse avec une liqueur basique pendant la séquence de nettoyage, soit avec la même liqueur basique EB que celle préparée dans la cuve 6, soit une liqueur basique différente, notamment en termes de concentration en base B. Cette variante permet de réduire la quantité de solution basique pure à introduire dans le réacteur de prétraitement 4, puisqu'il n'y aura plus, ou moins, d'acide à neutraliser pour arriver au pH basique visé. Cependant, une certaine quantité de liqueur basique sera éliminée dans le pressât (qui correspond à l'eau extraite de la vis 2' )

Ainsi, il peut rester nécessaire de faire un appoint de liqueur basique EB directement dans le réacteur 4 (via la cuve 51) : la cuve 51 de préparation de liqueur basique EB a toujours deux entrées, une pour la base B concentrée (KOH concentré), l'autre pour l'eau, mais ici elle a aussi deux sorties : une sortie vers le réacteur de prétraitement comme précédemment et une sortie vers le réacteur d'imprégnation 3. Avec cette configuration, on peut alimenter le réacteur d'imprégnation 3 soit avec la solution acide EA depuis la cuve 31 pendant la séquence T1, soit avec la solution basique EB depuis la cuve 6 pendant la séquence T2. La cuve 6 peut ainsi alimenter simultanément les deux réacteurs 3 et 4, ou au moins pendant une période commune pendant le nettoyage du réacteur 4. Il est également possible d'anticiper et de commencer à alimenter en solution basique EB l'un des réacteurs avant l'autre, notamment le réacteur d'imprégnation 3 avant le démarrage du nettoyage par la solution EB du réacteur de prétraitement 4.

D- On peut aussi combiner les deux variantes précédentes, avec, à la fois, le recyclage du pressât en sortie de la vis 2b dans la cuve 61 de préparation de liqueur basique EB, et l'alimentation par cette même cuve 51 des deux réacteurs 3 et 4 pendant au moins une partie de la séquence T2.

E- On peut aussi utiliser deux réacteurs d'imprégnation 3, 3' fonctionnant en alternance. Comme dans la variante C, on imprègne la biomasse non pas avec une liqueur acide EA mais avec une liqueur basique EB pendant au moins une partie du nettoyage/de la séquence T2 du réacteur, voire également un peu avant, de la façon suivante : en mode production, la biomasse est amenée dans le réacteur 3 de prétraitement alimentée en liqueur acide par la cuve 2, et en mode production + nettoyage (pendant tout ou partie du nettoyage), la biomasse est re-routée vers l'imprégnateur 3', qui, lui, est alimenté en liqueur basique EB depuis la cuve 51. On utilise donc un second réacteur d'imprégnation 3' dédié pour le nettoyage. Ce mode de réalisation présente l'avantage, par rapport à la variante C, de réduire les temps de transition entre imprégnations acides et imprégnations basiques.

F- On peut combiner les variantes E et B, c'est-à-dire utiliser les deux réacteurs d'imprégnation 3 ;3' et recycler l'eau extraite de la vis 2' dans la cuve 6 de préparation de liqueur basique EB.

G- On peut recycler le moût (appelé aussi marc), notamment dans le contexte de la variante E à deux réacteurs d'imprégnation : la biomasse prétraitée basique M1 qui sort du dispositif de séparation 8 pendant le nettoyage du réacteur de prétraitement 4. En effet, pendant cette période, il est basique. On peut alors laver ce mout MB en sortie du dispositif de séparation 8 avec de l'eau, il devient un mout basique lavé MB', et en extraire une phase aqueuse basique E1 qui est recyclée dans la cuve 51 de préparation de la liqueur basique.

H- Une autre variante consiste à utiliser deux dispositifs de séparation 5,5' (cyclone) fonctionnant en alternance : on ajoute un cyclone qui est dédié au traitement du marc basique MB. En mode de production/séquence T1, le premier cyclone est opérationnel, il traite un marc acide MA, en mode production + nettoyage/séquence T2, on bascule la sortie du réacteur 4 vers le second cyclone' qui ne séparera donc que du marc basique MB. L'avantage de cette variante est de réduire le temps de transition entre les deux modes. On peut aussi combiner cette variante avec la variante G : on lave également le marc basique MB une fois séparé dans un des cyclones pour recycler l'eau de lavage basique E1 vers la cuve de préparation 51 de liqueur basique.

J- Cette variante découle de la variante E à deux réacteurs d'imprégnation 3,3' précédentes, avec la différence suivante : En mode production, on utilise le réacteur d'imprégnation 3 conventionnel alimenté en solution acide EA par la cuve 1. En mode production + nettoyage, on bascule ici sur le second réacteur d'imprégnation 3' qui n'est alimenté qu'en eau : pendant le nettoyage/ la séquence T2, la biomasse n'est donc imprégnée que par une solution aqueuse à pH neutre (et non pas une solution basique EB).

K- L'invention s'applique également à des procédés de prétraitement de biomasse sans pré-imprégnation préalable avec une liqueur (on parle alors d'auto-hydrolyse) : dans ce cas, la biomasse P, après avoir éventuellement subi un traitement de type mécanique (broyage...), thermique (séchage), ou humidification, est directement introduite dans le réacteur de prétraitement 4.

L- Cette variante combine le recyclage du pressât E1 de la variante E avec celui de l'eau de lavage de la vis 2b de la variante B vers la cuve 6 de préparation de la liqueur basique EB. On diminue ainsi plus sensiblement à la fois la consommation en eau et en base nécessaire au nettoyage selon l'invention.

M- Cette variante préconise une intégration thermique du procédé, par condensation de la vapeur V en sortie du cyclone 8. Cette vapeur V sert à chauffer la liqueur basique EB circulant dans des conduites entre la cuve 51 et le réacteur de prétraitement 4 via un échangeur thermique (non représenté). Elle sert également à réduire la quantité d'eau utilisée dans la cuve 6 en récupérant le condensat issu du refroidissement de la vapeur sortant du cyclone, via un condenseur (non représenté).

N- Selon une autre variante, on peut choisir d'injecter de la biomasse imprégnée de liqueur acide EA dans le réacteur de prétraitement 4 depuis le réacteur d'imprégnation 3 en mode production, et injecter directement la biomasse P non imprégnée dans le réacteur de prétraitement 4 en séquence T2 (mode production + nettoyage), en stoppant alors l'alimentation en biomasse imprégnée de liqueur acide.

O- Selon encore une autre variante, cumulable avec toutes les autres, on peut choisir d'injecter dans le réacteur de prétraitement une biomasse imprégnée donnée en mode production (séquence T1), et d'injecter une autre biomasse, imprégnée ou non d'une liqueur, en mode production + nettoyage (séquence T2). Par exemple, en production, on choisit une biomasse de type paille, et en mode production + nettoyage, on choisit une biomasse plus abrasive, à base de peuplier : on augmente ainsi temporairement, le temps du nettoyage, le caractère abrasif de la biomasse, pour aider à décoller plus facilement les résidus solides de coke des parois.

### Exemples

### Exemple 1 non conforme à l'invention : utilisation d'un marc basique MB seul en SSCF

(« Simultaneous saccharification and co-fermentation » en anglais, c'est-à-dire hydrolyse enzymatique et fermentation alcoolique concomitantes)

Un lot de paille de blé est prétraité par explosion à la vapeur en conditions acides. Pour ce faire, la paille est broyée à 50 mm puis introduite dans un premier réacteur d'imprégnation 3. Les caractéristiques et composition de la paille de blé sont les suivantes :

| | |
|---|---|
| Matière sèche | : 91,07 % |
| Débit de biomasse | : 65 kg MS/h |

La paille est introduire dans un premier réacteur d'imprégnateur 3 afin d'être contactée avec une liqueur acide avant d'être envoyée dans un réacteur de prétraitement 4 par explosion à la vapeur. Les conditions opératoires en cours de production (séquence T1) sont les suivantes :
- Imprégnation :
   Débit de solution acide concentré: 1,29 kg/h, soit un pH d'environ 1
   Acidité de la liqueur acide : 1,1 g H₂SO₄ / 100g
   Temps d'imprégnation : 1 min
- Explosion Vapeur (réacteur de cuisson 4) :
   Temps de séjour : 5 min
   Durée de production : 20 heures

Après une période de 20 heures de fonctionnement dans ces conditions, une séquence T2 de nettoyage du réacteur 5 de prétraitement par explosion à la vapeur est enclenchée :
- Imprégnation:
   Débit de solution acide concentré : 1,29 kg/h, soit un pH d'environ 1
   Acidité de la liqueur acide: 1,1 g H₂SO₄ / 100g
   Temps d'imprégnation : 1 min
- Explosion Vapeur Séquence T2/Nettoyage :
   Temps de séjour : 10 min

Débit liqueur basique : suffisant afin de faire baisser la MS jusqu'à la valeur de saturation de la biomasse Recyclage du pressât (mélange d'eau de lavage du moyen d'alimentation du réacteur de cuisson 5 (« plug screw » 2b) et de la liqueur acide usé) : 100%
Durée du nettoyage/de la séquence T2 : 2 h
Concentration du KOH dans la liqueur basique : suffisante afin de passer la biomasse d'un pH de 3 à un pH de 13

Après la séquence de nettoyage T2, un cycle de 20 h de production T1 et 2 h de nettoyage T2 a été réalisé. Le nombre total de cycles réalisé est de 3 cycles de production/nettoyage (séquence T1/séquence T2).

Au total, 54 kgMS/h (durée de fonctionnement = 66 h ; m (paille) = 3540 kg) de paille de blé ont été consommés pour produire :
50 kgMS/h de substrat prétraité en conditions acides (durée de fonctionnement = 60 h, m(substrat acide) = 3029 kg)
78 kgMS/h de substrat prétraité en conditions alcalines (durée de fonctionnement = 4,5 h, m(substrat basique) = 353 kg)
70 kgMS/h de marcs « de transition » traités dans des conditions de pH variables au cours de la cuisson (durée de fonctionnement = 1,5 h ; m(substrat transition) = 106 kg)

Les marcs traités en conditions acides MA et en conditions alcalines MB sont échantillonnés. Des tests de SSCF sont conduits au laboratoire avec :
- Un test SSCF alimenté uniquement par du marc MA produit en conditions acides, avec une MS (matière sèche) finale de 24% de marc
- Un test SSCF alimenté uniquement par du marc MB produit en conditions alcalines, MS finale 19% de marc.

Les conditions de SSCF sont conventionnelles, et ne seront pas détaillées plus avant, elles comprennent une neutralisation pour atteindre le pH voulu pour maximiser l'activité des enzymes. Le test est réalisé en batch alimenté, une première partie du marc est mis en contact avec les biocatalyseurs, puis le reste du marc est ajouté pas à pas sur une durée de 6h afin de maitriser la viscosité du milieu dans les premières heures

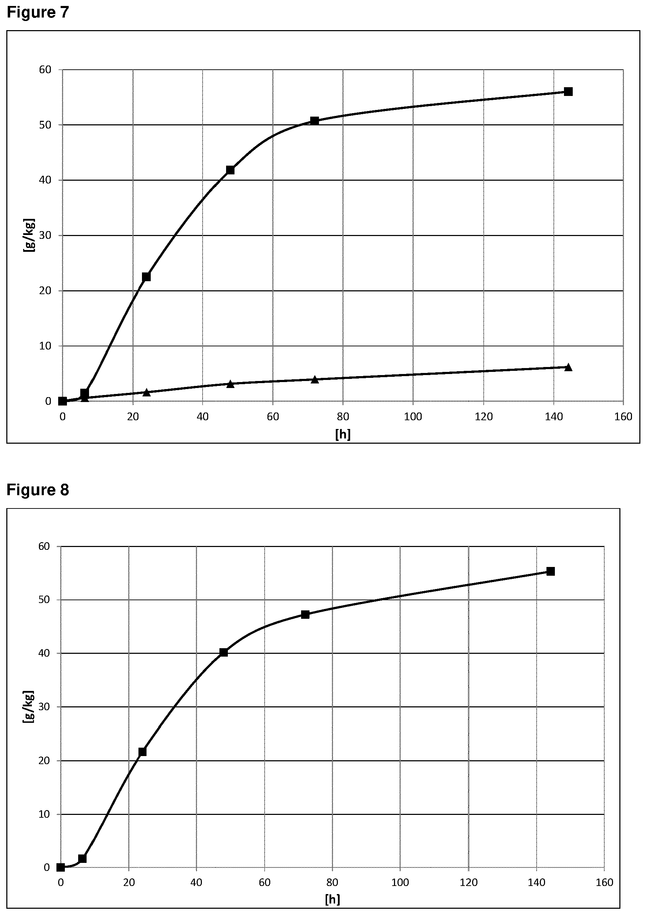

La figure 7 représente un graphe, avec, en abscisse, la durée de l'étape de SSCF en heure, et, en ordonnée, la quantité d'éthanol produite en g/kg La production d'éthanol issue du marc acide MA correspond à la courbe dont les points sont des losanges, la production d'éthanol issue du marc alcalin MB correspond à la courbe dont les points sont des carrés :
En exprimant ces résultats en concentration d'éthanol, on obtient :
- titre final du marc acide MA : 56 g / kg d'éthanol,
- titre final du marc alcalin MB : 6 g / kg d'éthanol.

En exprimant ces résultats en rendement de conversion, on obtient :
- sur marc acide, MA, on a un rendement 30.4%poids d'éthanol / sucres présents en entrée
- sur marc alcalin MB , ce rendement est de 7,5%.

Il apparait clairement, de la lecture de ce graphe et de ces données, que le marc alcalin utilisé seul MB ne permet pas d'obtenir une production satisfaisante d'éthanol.

### Exemple 2 : conforme à l'invention

Les marcs acides MA et alcalins MB produits à l'exemple 1 sont utilisés en mélange dans le test de SSCF selon le même protocole que pour l'exemple 1. Le test est alimenté par un mélange MA + MB de marcs acide et alcalin réalisé dans une proportion massique de 80% de marc acide MA et 20% de marc alcalin MB (poids brut). Ce mélange est réalisé avant l'introduction dans le réacteur 16/18 de SSCF. La MS finale introduite est de 24%MS provenant du mélange de marcs. L'alimentation en marc(s) se fait en partie dans une phase batch initiale, et en partie en fed-batch durant les 6 premières heures de réaction.

La cinétique de production d'éthanol est représentée dans le graphe de la figure 8, qui représente les mêmes données en abscisse et en ordonnée qu'au graphe de la figure 7, la courbe correspondant cette fois à la production d'éthanol à partir du mélange de marcs MA + MB.

On obtient :
- un titre final de 55,3 g / kg d'éthanol, ce qui est similaire aux résultats obtenus avec le marc acide MA seul on considère qu'un écart inférieur à 1 g / kg d'éthanol est non-significatif car dans la précision du test).
- un rendement de 32,7%pds d'éthanol / sucres présents en entrée

A noter qu'il a été nécessaire de réajuster le pH de ce mélange avec de l'acide sulfurique lors de la phase initiale de batch et de la phase de fed-batch.

Cet exemple démontre donc qu'avec une proportion significative (20%) en marc alcalin dans le mélanges de marcs, on peut continuer à obtenir une production d'éthanol sensiblement identique à celle obtenue avec un marc 100% acide : on peut donc procéder au nettoyage en continu du réacteur d'imprégnation sans impacter le rendement en éthanol de toute la production de l'installation, en utilisant tout le marc obtenu après prétraitement, qu'il soit acide ou alcalin, moyennant un mélange préalable des deux types de marcs.

### Exemple 3 conforme à l'invention : utilisation d'un marc acide MA, d'un marc alcalin MB et d'un marc lavé M1 issu d'un substrat bois de peuplier TCR (Taillis à Croissance Rapide)

Un lot de bois de peuplier TCR est prétraité par explosion à la vapeur en conditions acides. Pour ce faire, le bois de peuplier TCR est broyé à 50 mm, puis introduit dans un premier réacteur d'imprégnation 3.

Les caractéristiques et composition du bois sont les suivantes :
Matière sèche MS : 55,50 %poids
Débit de fonctionnement : 64 kg MS/h

Le bois est introduit dans un premier réacteur d'imprégnateur 3 afin d'être contacté avec une liqueur acide avant d'être envoyée dans un réacteur 5 de prétraitement par explosion à la vapeur. Les conditions opératoires en cours de production sont les suivantes :
- Imprégnation pour la production et le nettoyage (séquences T1 et T2) :
   Débit de solution acide concentré: 2,6 kg/h
   Conductivité de la liqueur acide à 80°C : 103 mS/cm
   Temps d'imprégnation : 1 min
- Explosion Vapeur (séquence T1/Production) :
   Temps de séjour : 7,5 min
   Alimentation vapeur : 100% en tête
   Recyclage pressât : 100%
   Durée de production : 58.5 heures

Après une période de 60 heures de fonctionnement dans ces conditions, une séquence de nettoyage / séquence T2 du réacteur 5 d'explosion à la vapeur est enclenchée :
- Imprégnation pour la production et le nettoyage/séquence T2 :
   Débit de solution acide concentré : 2,6 kg/h
   Conductivité de la liqueur acide à 80°C : 103 mS/cm
- Explosion Vapeur Nettoyage / séquence T2 :
   Temps de séjour : 10 min
   Débit liqueur basique : suffisant afin de faire baisser la MS jusqu'à la valeur de saturation de la biomasse
   Recyclage pressât : 100%
   Durée du nettoyage : 1,5 h
   Concentration du KOH dans la liqueur : suffisante afin de passer la biomasse d'un pH 3 à un pH 13
   Nombre de cycles : 3 cycles de production et de nettoyage (3 x séquence T1 + séquence T2)

Au total, 116 kg/h (durée de fonctionnement = 60 h ; m(paille) = 6960 kg) de peuplier TCR ont été consommés pour produire :
119 kg/h de substrat prétraité en conditions acides (durée fonctionnement = 58,5 h, m(substrat acide) = 6962 kg)
361 kg/h de substrat prétraité en conditions alcalines (durée fonctionnement = 1 h, m (substrat basique) = 361 kg)
178 kg/h de marcs de transition traités dans des conditions de pH variables au cours de la cuisson (durée fonctionnement = 30 min ; m(substrat transition) = 89 kg)

Une partie du marc traité en condition acide est filtré sur un filtre à bande, par un lavage à contre-courant, pour séparer un jus sucré (servant à la propagation de levures), d'un marc partiellement lavé nommé M1 qui sera réintroduit en liquéfaction.

Des tests de SSCF sont conduits sur les marcs prétraités en conditions acides, en conditions alcalines, et sur les marcs partiellement lavés (les marcs de transition n'ont pas été différenciés ici):
Un test SSCF par du marc MA produit en conditions acides, du marc produit en conditions alcalines MB , du marc M1 prétraité partiellement lavé, 20 mg d'enzymes/g MS, pour une MS finale de 22,2%poids. Cet essai a nécessité 23,7 kg de solution de neutralisation (à 2,.5%pds en NH₃).

Le protocole de la SSCF commence par une étape dite de liquéfaction réalisée en fed-batch

Le mélange initial de réaction est composé d'eau, du marc basique, du marc partiellement lavé et d'une partie du marc produit en conditions acides (jusqu'à 12,5%MS en pied de cuve) et complémentés en nutriments pour les levures. Le pH est ajusté à 5,3 avec une solution alcaline (contenant 23,5%poids de NH3). Lorsque le pH est à 5,3, les biocatalyseurs enzymes et levures sont ajoutés. Cet ajout correspond au démarrage de la réaction d'hydrolyse enzymatique, et de la réaction de fermentation concomitante. Après un temps court, l'ajout en fed-batch du reste du marc produit en conditions acides est réalisé sur une durée de 2heures

Lorsque la rhéologie du mélange le permet, le mélange est transféré dans une cuve de SSCF où les réactions d'hydrolyse et de fermentation se poursuivent. La durée totale de la SSCF est de 144 h (total dans les deux réacteurs)

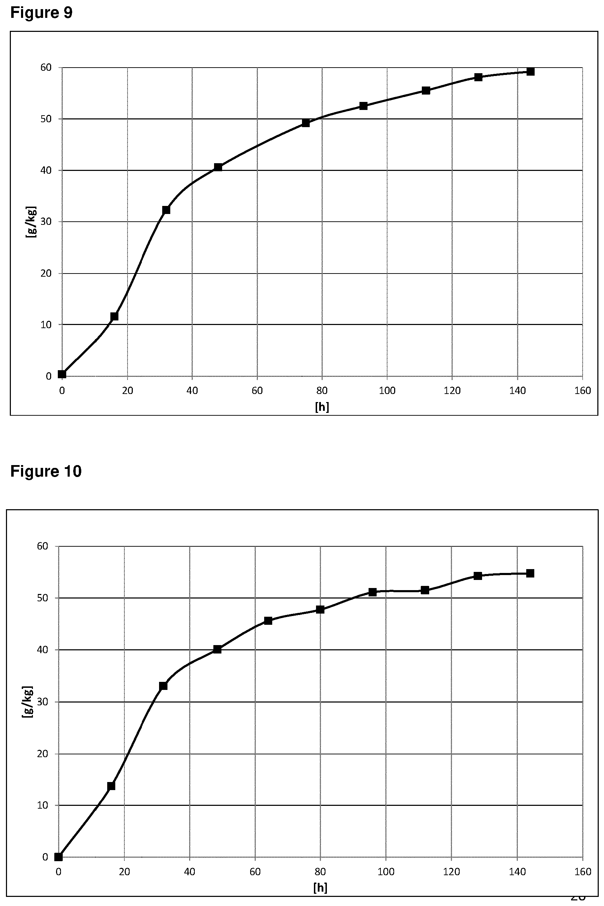

Le graphe de la figure 9, avec les mêmes conventions des graphes des figures précédentes, correspond à la production d'éthanol produite à parti de ce marc. Cette mise en œuvre permet :
- Pour la SSCF , un titre final de 59.2 g / kg d'éthanol
- Pour la SSCF, un rendement de 41.3%pds d'éthanol / sucres présents en entrée

### Exemple 4 conforme à l'invention : marc acide MA et marc alcalin MB issus d'un substrat paille.

Un lot de Paille de blé est prétraité par explosion à la vapeur en conditions acides. Pour ce faire, la paille est broyée à 50 mm, dépoussiérée, puis introduite dans un premier réacteur d'imprégnation.

Les caractéristiques et composition de la paille de blé sont les suivantes :
Matière sèche : 88,30 %poids
Débit de fonctionnement : 65 kg MS/h

La paille est introduire dans un premier réacteur d'imprégnateur 3 afin d'être contactée avec une liqueur acide avant d'être envoyée dans un réacteur de prétraitement 5 par explosion à la vapeur. Les conditions opératoires en cours de production sont les suivantes :
- Imprégnation pour la production et le nettoyage (séquences T1 et T2) :
   Débit de solution acide concentré : 2,5 kg/h
   Conductivité de la liqueur acide à 80°C : 103 mS/cm
   Temps d'imprégnation : 1 min
- Explosion Vapeur Production (séquence T1) .
   Temps de séjour : 5 min
   Alimentation vapeur : 100% en tête
   Recyclage pressât : 100%
   Durée de production : 78 heures

Après une période de 80 heures de fonctionnement dans ces conditions, une séquence de nettoyage /T2 du réacteur 5 d'explosion à la vapeur est enclenchée :
- Imprégnation pour la production et le nettoyage (séquence T2) :
   Débit de solution acide : 2,5 kg/h
   Conductivité de la liqueur acide à 80°C : 103 mS/cm
- Explosion Vapeur Nettoyage / séquence T2:
   Temps de séjour : 10 min
   Débit liqueur basique : suffisant afin de faire baisser la MS jusqu'à la valeur de saturation de la biomasse
   Recyclage pressât : 100%
   Durée du nettoyage : 2 h
   Concentration du KOH dans la liqueur : suffisante afin de passer la biomasse d'un pH 3 à un pH 13
   Nombre de cycles : 4 cycles de production et de nettoyage : 4 cycles T1 + T2

Au total, durant les 4 cycles de production/nettoyage, 73 kg/h (durée fonctionnement = 320 h ; m(paille) = 23360 kg) de paille de blé ont été consommés pour produire :
138 kg/h de substrat prétraité en conditions acides (durée fonctionnement = 312 h, m(substrat acide) = 43056 kg)
342 kg/h de substrat prétraité en conditions alcalines (durée fonctionnement = 8 h, m(substrat basique) = 2732 kg)

Des tests de SSCF sont conduits sur les marcs MA prétraités en conditions acides et en conditions alcalines MB, sans différencier les marcs de transition :
Un test SSCF a été réalisé avec du marc MA produit en conditions acides, du marc MB produit en conditions alcalines, pour une MS finale de 22,5%poids. Cet essai a nécessité 42.7 kg de solution de neutralisation (à 23.5%pds en NH3).

Le protocole de la SSCF commence par une étape dite de liquéfaction pour arriver à un mélange 92%poids marc produit en conditions acides/8%poids marc produit en conditions basiques. La liquéfaction est opérée en fed-batch. Le mélange initial de réaction est composé d'eau, du marc basique, et d'une partie du marc produit en conditions acides (jusqu'à 12,5%MS en pied de cuve) et complémentés en nutriments pour les levures. Le pH est ajusté à 5,3 avec une solution alcaline (contenant 23,5%poids de NH₃). Lorsque le pH est à 5,3, les biocatalyseurs enzymes et levures sont ajoutés. Cet ajout correspond au démarrage de la réaction d'hydrolyse enzymatique, et de la réaction de fermentation concomitante. Après un temps court, l'ajout en fed-batch du reste du marc produit en conditions acides est réalisé sur une durée de 6heures

Lorsque la rhéologie du mélange le permet, le mélange est transféré dans une cuve de SSCF où les réactions d'hydrolyse et de fermentation se poursuivent. La durée totale de la SSCF est de 144 h (total dans les deux réacteurs).

La cinétique de production d'éthanol est décrite dans le graphe de la figure 10, avec les mêmes conventions que pour les graphes précédents.

Cette mise en œuvre conforme à l'invention permet d'obtenir :
- Un titre final de 54,8 g / kg d'éthanol
- Un rendement de 36,2 %pds d'éthanol / sucres présents en entrée

Avec un ajout de 8%poids brut de marc MB dans le marc acide MA, la double réaction de SSCF s'effectue donc de façon satisfaisante.

### Exemple 5 conforme à l'invention : marc acide MA, marc alcalin MB et marc lavé M1, issus d'un substrat paille (conditions de prétraitement et de lavage différentes de celles de l'exemple 4, avec une baisse de la température du réacteur)

Un lot de Paille de blé est prétraité par explosion à la vapeur en conditions acides. Pour ce faire, la paille est broyée à 50 mm, dépoussiérée, puis introduite dans un premier réacteur d'imprégnation 3.

Les caractéristiques et composition de la paille de blé sont les suivantes :
Matière sèche : 88,30 %poids
Débit de fonctionnement : 50 kg MS/h

La paille est introduire dans un premier réacteur d'imprégnation 3 afin d'être contacté avec une liqueur acide avant d'être envoyée dans un réacteur de prétraitement 5 par explosion à la vapeur. Les conditions opératoires en cours de production sont les suivantes :
- Imprégnation pour la production et le nettoyage (séquence T1) :
   Débit de solution acide concentré : 2,5 kg/h
   Conductivité de la liqueur acide à 80°C : 103 mS/cm
   Temps d'imprégnation : 1 min
- Explosion Vapeur en Production/séquence T1 :
   Temps de séjour : 5 min
   Alimentation vapeur : 100% en tête
   Recyclage pressât : 100%
   Durée de production : 78 heures

Après une période de 80 heures de fonctionnement dans ces conditions, une séquence de nettoyage T2 du réacteur 5 d'explosion à la vapeur est enclenchée :
- Imprégnation pour la production et le nettoyage /séquence T2 :
   Débit de solution acide: 2,5 kg/h
   Conductivité de la liqueur acide à 80°C : 103 mS/cm
- Explosion Vapeur Nettoyage / séquence T2 :
   Temps de séjour : 10 min
   Débit liqueur basique : suffisant afin de faire baisser la MS jusqu'à la valeur de saturation de la biomasse
   Recyclage pressât : 100%
   Durée du nettoyage : 2 h
   Concentration du KOH dans la liqueur : suffisante afin de passer la biomasse d'un pH 3 à un pH 13
   Nombre de cycles : 1 cycle de production et de nettoyage (T1 + T2)

Au total, durant ce cycle de production/nettoyage, 58 kg/h (durée de fonctionnement = 80 h ; m(paille) = 4640 kg) de paille de blé ont été consommés pour produire :
109 kg/h de substrat prétraité en conditions acides (durée de fonctionnement = 78 h, m(substrat acide) = 8502 kg)
372 kg/h de substrat prétraité en conditions alcalines (durée de fonctionnement = 2 h, m(substrat basique) = 744 kg)

Un test de SSCF a été conduit au pilote sur les marcs prétraités en conditions acides MA et en conditions alcalines MB, pour une MS finale de 22,5%poids. Cet essai a nécessité 33,8 kg de solution de neutralisation (à 23,5%pds en NH₃).

Le protocole de la SSCF commence par une étape dite de liquéfaction pour arriver à un mélange 90%poids marc produit en conditions acides MA /10%pds marc produit en conditions basiques MB.

La liquéfaction est opérée en fed-batch. Le mélange initial de réaction est composé d'eau, du marc basique, et d'une partie du marc produit en conditions acides (jusqu'à 12,5%MS en pied de cuve) et complémentés en nutriments pour les levures. Le pH est ajusté à 5,3 avec une solution alcaline (contenant 23.5%poids de NH₃). Lorsque le pH est à 5,3, les biocatalyseurs enzymes et levures sont ajoutés. Cet ajout correspond au démarrage de la réaction d'hydrolyse enzymatique, et de la réaction de fermentation concomitante. Après un temps court, l'ajout en fed-batch du reste du marc produit en conditions acides est réalisé sur une durée de 6heures

Lorsque la rhéologie du mélange le permet, le mélange est transféré dans une cuve de SSCF où les réactions d'hydrolyse et de fermentation se poursuivent. La durée totale de la SSCF est de 118h (total dans les deux réacteurs)

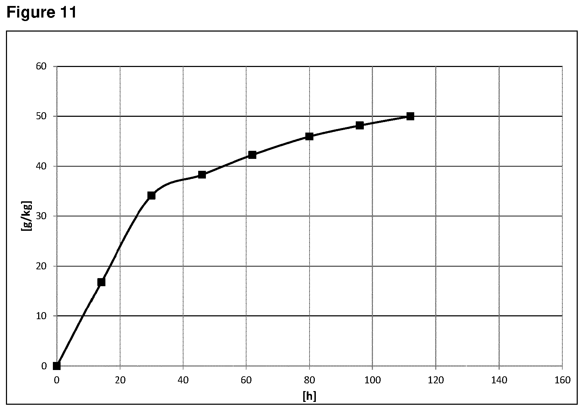

La cinétique de production d'éthanol est décrite dans le graphe de la figure 11, qui reprend les mêmes conventions que les graphes précédents.

Cette mise en œuvre conforme à l'invention permet, à 118 heures, d'obtenir :
- Un titre final de 50,0 g / kg d'éthanol
- Un rendement de 32,2 %pds d'éthanol / sucres présents en entrée

La production d'éthanol reste, là encore, à un niveau satisfaisant même avec ajout de marc basique en quantité significative (10%) au marc acide MA conventionnel.

## Revendications

1. Procédé de traitement de biomasse lignocellulosique comprenant, successivement,
b) une étape de prétraitement par cuisson, avec ou sans explosion à la vapeur de la biomasse préalablement mise dans des conditions acides ou à pH neutre dans un réacteur de prétraitement (3), pour produire un marc prétraité acide (MA) ou neutre,
en alternance avec
b') une étape de prétraitement par cuisson, avec ou sans explosion à la vapeur de la biomasse préalablement mise dans des conditions acide, neutre ou basique, avec introduction suffisante dans le réacteur de prétraitement (3) d'une solution aqueuse basique (EB) au moins pour la biomasse préalablement mise en condition acide ou neutre, pour produire un marc prétraité basique (MB),
puis
c) une étape d'hydrolyse enzymatique dans un réacteur d'hydrolyse (16) d'un mélange du marc prétraité acide (MA) ou neutre issu de l'étape b) avec le marc prétraité basique (MB) issu de l'étape b').

2. Procédé selon la revendication précédente, **caractérisé en ce que**, pendant l'étape c) d'hydrolyse enzymatique du mélange de marc prétraité acide (MA) ou neutre avec le marc prétraité basique (MB), on remplace ledit mélange par seulement du marc prétraité acide (MA) ou neutre pendant une partie seulement de la durée de ladite l'étape c).

3. Procédé selon l'une des revendications précédentes, **caractérisé en ce que** le mélange du marc prétraité acide (MA) ou neutre issu de l'étape b) avec le marc prétraité basique (MB) issu de l'étape b') dans l'étape c) est réalisé en amont du réacteur d'hydrolyse (16), ou directement dans le réacteur d'hydrolyse (16).

4. Procédé selon l'une des revendications précédentes, **caractérisé en ce qu'**on réalise le mélange, dans l'étape c), du marc prétraité acide (MA) ou neutre issu de l'étape b) avec le marc prétraité basique (MB) issu de l'étape b') par prélèvements à partir d'une zone de stockage temporaire (13) du marc prétraité basique (MB) en sortie du réacteur de prétraitement (5).

5. Procédé selon la revendication précédente, **caractérisé en ce que** l'étape b') produisant un marc prétraité basique (MB) est une étape de nettoyage du réacteur de prétraitement (5).

6. Procédé selon l'une des revendications précédentes, **caractérisé en ce qu'**on prévoit une étape d'ajustement du pH du marc prétraité (MA,MB) ou du mélange de marcs prétraités avant ou pendant l'étape c) d'hydrolyse enzymatique, notamment un ajustement du pH entre 4 et 6.

7. Procédé selon l'une des revendications précédentes, **caractérisé en ce que**, sur un temps de production donné, la somme des durées des étapes de prétraitement b) de la biomasse préalablement mise dans des conditions acides ou à pH neutre dans un réacteur de prétraitement, pour produire un marc prétraité acide (MA) ou neutre, est supérieure, notamment au moins 2 à 5 fois supérieure, à la somme des durées des étapes b') de prétraitement de la biomasse préalablement mise dans des conditions acide, neutre ou basique, avec éventuelle introduction suffisante dans le réacteur de prétraitement d'une solution aqueuse basique (EB), pour produire un marc prétraité basique (MB).

8. Procédé selon l'une des revendications précédentes, **caractérisé en ce qu'**on réalise une séparation entre marc prétraité (MA,MB) et phase aqueuse sous forme liquide ou vapeur en sortie du réacteur de prétraitement (5) par un dispositif de séparation (8) ou plusieurs dispositifs de séparation en parallèle, notamment deux dispositifs de séparation, fonctionnant en alternance.

9. Procédé selon l'une des revendications précédentes, **caractérisé en ce que**, lors de l'étape c) d'hydrolyse enzymatique dans le réacteur d'hydrolyse (16), le mélange du marc (MA) prétraité acide ou neutre issu de l'étape b) avec le marc prétraité (MB) basique issu de l'étape b') se fait dans un ratio MA/MB en poids d'au moins 80/20.

10. Procédé selon l'une des revendications précédentes, **caractérisé en ce que** l'étape de prétraitement b) est précédé d'une étape a) d'imprégnation dans un réacteur d'imprégnation (3) de la biomasse par une solution aqueuse acide ou neutre.

11. Installation de traitement de biomasse lignocellulosique, comprenant d'amont en aval un réacteur (3) d'imprégnation de biomasse en connexion fluidique avec une cuve (31) de préparation de solution aqueuse acide, un réacteur de prétraitement (5) de la biomasse imprégnée en connexion fluidique avec une cuve (51) de préparation d'une solution aqueuse basique, un dispositif de séparation (8) du marc prétraité en aval du réacteur de prétraitement (5) et qui est éventuellement associé à des moyens de rinçage par une solution aqueuse (E), et qui est en connexion fluidique avec une zone de stockage intermédiaire (13) de marc basique(MB), et éventuellement une zone de stockage intermédiaire (11) de marc acide (MA), et un réacteur d'hydrolyse enzymatique (16) alimenté en marcs depuis la/ l'une au moins desdites zones de stockage (11,13).

12. Installation selon la revendication précédente, **caractérisée en ce qu'**elle comprend une zone d'ajustement (14) du pH des marcs prétraités entre la/au moins une des zones de stockage (11,13) et le réacteur d'hydrolyse enzymatique (16), notamment une zone d'ajustement du pH commune pour lesdits marcs.

13. Installation selon la revendication précédente, **caractérisé en ce que** la zone d'ajustement est une cuve ou une trémie assurant le mélange des marcs issus d'au moins la ou une des zones de stockage (11,13).

14. Installation selon l'une des revendications 11 à 13, **caractérisée en ce qu'**elle comprend deux réacteurs d'imprégnation (3) et/ou deux dispositifs de séparation (8) et/ou deux réacteurs de prétraitement (5) fonctionnant en alternance selon que le prétraitement est opéré sur de la biomasse préalablement mise dans des conditions acides ou à pH neutre pour produire un marc prétraité acide(MA) ou neutre, selon l'étape b), ou sur de la biomasse préalablement mise dans des conditions acide, neutre ou basique, avec introduction suffisante dans le réacteur de prétraitement d'une solution aqueuse basique (EB), pour produire un marc prétraité basique (MB) selon l'étape b').

15. Utilisation du procédé ou de l'installation selon les revendications précédentes, pour le traitement de biomasses du type bois, paille, résidus agricoles, et toutes cultures énergétiques dédiées, notamment des plantes annuelles ou pluriannuelles telles que le miscanthus, en vue de produire des sucres, des biocarburants ou des molécules bio-sourcées.

## Patentansprüche

1. Verfahren zur Behandlung von lignocellulosehaltiger Biomasse, umfassend, nacheinander,
b) einen Vorbehandlungsschritt durch Kochen mit oder ohne Dampfexplosion der zuvor in saure oder pH-neutrale Bedingungen versetzten Biomasse in einem Vorbehandlungsreaktor (3), um einen sauren oder neutralen vorbehandelten Trester (MA) zu erzeugen,
im Wechsel mit
b') einem Vorbehandlungsschritt durch Kochen mit oder ohne Dampfexplosion der zuvor in saure, neutrale oder basische Bedingungen versetzten Biomasse mit ausreichender Einleitung einer basischen wässrigen Lösung (EB) in den Vorbehandlungsreaktor (3) mindestens für die zuvor in saure oder neutrale Bedingungen versetzte Biomasse, um einen basischen vorbehandelten Trester (MB) zu erzeugen,
dann
c) einen Schritt der enzymatischen Hydrolyse in einem Hydrolysereaktor (16) einer Mischung des aus dem Schritt b) hervorgegangenen sauren oder neutralen vorbehandelten Tresters (MA) mit dem aus dem Schritt b') hervorgegangenen basischen vorbehandelten Trester (MB).

2. Verfahren nach dem vorhergehenden Anspruch, **dadurch gekennzeichnet, dass** während des Schritts c) der enzymatischen Hydrolyse der Mischung des sauren oder neutralen vorbehandelten Tresters (MA) mit dem basischen vorbehandelten Trester (MB) das Mischung durch nur sauren oder neutralen vorbehandelten Trester (MA) während nur eines Teils der Dauer des Schritts c) ersetzt wird.

3. Verfahren nach einem der vorhergehenden Ansprüche, **dadurch gekennzeichnet, dass** das Mischen des aus dem Schritt b) hervorgegangenen sauren oder neutralen vorbehandelten Tresters (MA) mit dem aus dem Schritt b') hervorgegangenen basischen vorhandelten Trester (MB) in dem Schritt c) stromauf des Hydrolysereaktors (16) oder direkt in dem Hydrolysereaktor (16) ausgeführt wird.

4. Verfahren nach einem der vorhergehenden Ansprüche, **dadurch gekennzeichnet, dass** das Mischung, in dem Schritt c), des aus dem Schritt b) hervorgegangenen sauren oder neutralen vorbehandelten Tresters (MA) mit dem aus dem Schritt b') hervorgegangenen basischen vorbehandelten Trester (MB) durch Entnahmen aus einem Bereich zur vorübergehenden Lagerung (13) des aus dem Vorbehandlungsreaktor (5) ausgetretenen basischen vorbehandelten Tresters (MB) ausgeführt wird.

5. Verfahren nach dem vorhergehenden Anspruch, **dadurch gekennzeichnet, dass** der Schritt b'), der einen basischen vorbehandelten Trester (MB) erzeugt, ein Schritt zum Reinigen des Vorbehandlungsreaktors (5) ist.

6. Verfahren nach einem der vorhergehenden Ansprüche, **dadurch gekennzeichnet, dass** ein Schritt des Einstellens des pH-Werts des vorbehandelten Tresters (MA,MB) oder der Mischung aus vorbehandelten Trestern vor oder während des Schritts c) der enzymatischen Hydrolyse vorgesehen wird, insbesondere ein Einstellen des pH-Werts zwischen 4 und 6.

7. Verfahren nach einem der vorhergehenden Ansprüche, **dadurch gekennzeichnet, dass**, über eine gegebene Produktionszeit, die Summe der Dauern der Vorbehandlungsschritte b) der zuvor in saure oder pH-neutrale Bedingungen versetzten Biomasse in einem Vorbehandlungsreaktor, um einen sauren oder neutralen vorbehandelten Trester (MA) zu erzeugen, größer, insbesondere mindestens 2 bis 5 Mal größer, ist als die Summe der Dauern der Vorbehandlungsschritte b') der zuvor in saure, neutrale oder basische Bedingungen versetzten Biomasse mit etwaiger ausreichender Einleitung in den Vorbehandlungsreaktor einer basischen wässrigen Lösung (EB), um einen basischen vorbehandelten Trester (MB) zu erzeugen.

8. Verfahren nach einem der vorhergehenden Ansprüche, **dadurch gekennzeichnet, dass** eine Trennung zwischen vorbehandeltem Trester (MA,MB) und wässriger Phase in flüssiger oder Dampfform im Ausgang des Vorbehandlungsreaktors (5) durch eine Trennvorrichtung (8) oder mehrere parallele Trennvorrichtungen, insbesondere zwei im Wechsel betriebene Trennvorrichtungen, ausgeführt wird.

9. Verfahren nach einem der vorhergehenden Ansprüche, **dadurch gekennzeichnet, dass** bei dem Schritt c) der enzymatischen Hydrolyse in dem Hydrolysereaktor (16) das Mischen des aus dem Schritt b) hervorgegangenen sauren oder neutralen vorbehandelten Tresters (MA) mit dem aus dem Schritt b') hervorgegangenen basischen vorbehandelten Trester (MB) in einem Gewichtsverhältnis MA/MB von mindestens 80/20 erfolgt.

10. Verfahren nach einem der vorhergehenden Ansprüche, **dadurch gekennzeichnet, dass** dem Vorbehandlungsschritt b) ein Schritt a) des Imprägnierens der Biomasse mit einer sauren oder neutralen wässrigen Lösung in einem Imprägnierreaktor (3) vorausgeht.

11. Anlage zur Behandlung von lignocellulosehaltiger Biomasse, umfassend von stromauf nach stromab einen Imprägnierreaktor (3) zum Imprägnieren von Biomasse in Fluidverbindung mit einem Behälter (31) zur Zubereitung einer sauren wässrigen Lösung, einen Vorbehandlungsreaktor (5) zum Vorbehandeln der imprägnierten Biomasse in Fluidverbindung mit einem Behälter (51) zur Zubereitung einer basischen wässrigen Lösung, eine Trennvorrichtung (8) zum Trennen des vorbehandelten Tresters stromab des Vorbehandlungsreaktors (5) und die gegebenenfalls Mittel zum Spülen mit einer wässrigen Lösung (E) zugeordnet ist, und die in Fluidverbindung mit einem Bereich zur Zwischenlagerung (13) von basischem Trester (MB) und gegebenenfalls einem Bereich zur Zwischenlagerung (11) von saurem Trester (MA) steht, und einen Reaktor zur enzymatischen Hydrolyse (16), dem Trester von dem/mindestens einem der Lagerbereiche (11,13) zugeführt werden.

12. Anlage nach dem vorhergehenden Anspruch, **dadurch gekennzeichnet, dass** sie einen Einstellbereich (14) zum Einstellen des pH-Werts der vorbehandelten Trester zwischen dem/mindestens einem der Lagerbereiche (11,13) und dem Reaktor zur enzymatische Hydrolyse (16) umfasst, insbesondere einen gemeinsamen Bereich zum Einstellen des pH-Werts für die Trester.

13. Anlage nach dem vorhergehenden Anspruch, **dadurch gekennzeichnet, dass** der Einstellbereich ein Behälter oder ein Bunker ist, der das Mischen der aus mindestens dem oder einem der Lagerbereiche (11,13) hervorgegangenen Trester gewährleistet.

14. Anlage nach einem der Ansprüche 11 bis 13, **dadurch gekennzeichnet, dass** sie zwei Imprägnierreaktoren (3) und/oder zwei Trennvorrichtungen (8) und/oder zwei Vorbehandlungsreaktoren (5) umfasst, die im Wechsel betrieben werden, je nachdem, ob die Vorbehandlung gemäß dem Schritt b) an zuvor in saure oder pH-neutrale Bedingungen versetzter Biomasse vorgenommen wird, um einen sauren oder neutralen vorbehandelten Trester zu erzeugen, oder gemäß dem Schritt b') an zuvor in saure, neutrale oder basische Bedingungen versetzter Biomasse, mit ausreichender Einleitung in den Vorbehandlungsreaktor einer basischen wässrigen Lösung (EB), um einen basischen vorbehandelten Trester (MB) zu erzeugen.

15. Verwendung des Verfahrens oder der Anlage nach den vorhergehenden Ansprüchen zur Behandlung von Biomassen vom Typ Holz, Stroh, landwirtschaftliche Reststoffe und dedizierte Energiepflanzen, insbesondere ein- oder mehrjährige Pflanzen wie Miscanthus, zur Erzeugung von Zucker, Biokraftstoffen oder biobasierten Molekülen.

## Claims

1. Process for treating lignocellulosic biomass, successively comprising
b) a step of pretreatment by cooking, with or without steam explosion, of the biomass placed beforehand under acidic or neutral pH conditions in a pretreatment reactor (3), to produce an acidic or neutral pretreated marc (AM),
alternating with
b') a step of pretreatment by cooking, with or without steam explosion, of the biomass placed beforehand under acidic, neutral or basic conditions, with sufficient introduction into the pretreatment reactor (3) of a basic aqueous solution (EB), at least for the biomass placed beforehand under acidic or neutral conditions, to produce a basic pretreated marc (BM),
and then
c) a step of enzymatic hydrolysis in a hydrolysis reactor (16) of a mixture of the acidic or neutral pretreated marc (AM) obtained from step b) with the basic pretreated marc (BM) obtained from step b').

2. Process according to the preceding claim, **characterized in that**, during step c) of enzymatic hydrolysis of the mixture of acidic or neutral pretreated marc (AM) with the basic pretreated marc (BM), said mixture is replaced with only acidic or neutral pretreated marc (AM) for a portion only of the duration of said step c).

3. Process according to either of the preceding claims, **characterized in that** the mixing of the acidic or neutral pretreated marc (AM) obtained from step b) with the basic pretreated marc (BM) obtained from step b') in step c) is performed upstream of the hydrolysis reactor (16), or directly in the hydrolysis reactor (16).

4. Process according to one of the preceding claims, **characterized in that** the mixing, in step c), of the acidic or neutral pretreated marc (AM) obtained from step b) with the basic pretreated marc (BM) obtained from step b') is performed by withdrawals from a zone for temporary storage (13) of the basic pretreated marc (BM) at the outlet of the pretreatment reactor (5).

5. Process according to the preceding claim, **characterized in that** step b') producing a basic pretreated marc (BM) is a step of cleaning the pretreatment reactor (5).

6. Process according to one of the preceding claims, **characterized in that** a step of adjusting the pH of the pretreated marc (AM, BM) or of the mixture of pretreated marcs before or during the enzymatic hydrolysis step c), notably an adjustment of the pH to between 4 and 6, is envisaged.

7. Process according to one of the preceding claims, **characterized in that**, over a given production time, the sum of the durations of the steps b) of pretreatment of the biomass placed beforehand under acidic or neutral pH conditions in a pretreatment reactor, to produce an acidic or neutral pretreated marc (AM), is greater, notably at least 2 to 5 times greater, than the sum of the durations of the steps b') of pretreatment of the biomass placed beforehand under acidic, neutral or basic conditions, with optional sufficient introduction into the pretreatment reactor of a basic aqueous solution (EB), to produce a basic pretreated marc (BM).

8. Process according to one of the preceding claims, **characterized in that** a separation is performed between pretreated marc (AM, BM) and aqueous phase in the liquid or vapour form at the outlet of the pretreatment reactor (5), by a separation device (8) or several separation devices in parallel, notably two separation devices, operating alternately.

9. Process according to one of the preceding claims, **characterized in that**, during the enzymatic hydrolysis step c) in the hydrolysis reactor (16), the mixing of the acidic or neutral pretreated marc (AM) obtained from step b) with the basic pretreated marc (BM) obtained from step b') is performed in an AM/BM weight ratio of at least 80/20.

10. Process according to one of the preceding claims, **characterized in that** the pretreatment step b) is preceded by a step a) of impregnating the biomass with an acidic or neutral aqueous solution, in an impregnation reactor (3).

11. Facility for treating lignocellulosic biomass, comprising, from upstream to downstream, a biomass impregnation reactor (3) in fluid connection with a vessel (31) for preparation of acidic aqueous solution, a reactor for pretreatment (5) of the impregnated biomass, in fluid connection with a vessel (51) for preparation of a basic aqueous solution, a pretreated marc separation device (8) downstream of the pretreatment reactor (5) and which is optionally combined with means for rinsing with an aqueous solution (E), and which is in fluid connection with a zone for intermediate storage (13) of basic marc (BM), and optionally a zone for intermediate storage (11) of acidic marc (AM), and an enzymatic hydrolysis reactor (16) fed with marcs from said/at least one of said storage zones (11, 13).

12. Facility according to the preceding claim, **characterized in that** it comprises a zone (14) for adjusting the pH of the pretreated marcs between the/at least one of the storage zones (11, 13) and the enzymatic hydrolysis reactor (16), notably a pH adjustment zone that is common for said marcs.

13. Facility according to the preceding claim, **characterized in that** the adjustment zone is a vessel or a hopper which ensures the mixing of the marcs obtained from at least the or one of the storage zones (11, 13).

14. Facility according to one of Claims 11 to 13, **characterized in that** it comprises two impregnation reactors (3) and/or two separation devices (8) and/or two pretreatment reactors (5) operating alternately depending on whether the pretreatment is performed on biomass placed beforehand under acidic or neutral pH conditions to produce an acidic or neutral pretreated marc (AM), according to step b), or on biomass placed beforehand under acidic, neutral or basic conditions, with sufficient introduction into the pretreatment reactor of a basic aqueous solution (EB) to produce a basic pretreated marc (BM) according to step b').

15. The use of the process or of the facility according to the preceding claims, for the treatment of biomasses such as wood, straw, agricultural residues, and all dedicated energy crops, notably annual or perennial plants such as miscanthus, for the purpose of producing sugars, biofuels or biobased molecules.
